# EUROPEAN PATENT APPLICATION

(11) **EP 4 810 497 A1**
(43) Date of publication of application: **23.09.2026**
(21) Application number: 24905332.3
(22) Date of filing: 15.03.2024
(51) Int. Cl.: C07D 491/056, A61K 31/496, A61P 3/00, A61P 3/10, A61P 3/04, A61P 25/28

(54) **ISOQUINOLINE DERIVATIVE, SYNTHESIS METHOD THEREFOR, AND USE THEREOF**

(30) Priority: 20.12.2023 CN 202311753379
(71) Applicant: Harbin Gloria Pharmaceuticals Co., Ltd., Harbin, Heilongjiang 150025 (CN)
(72) Inventor: LIU, Lei, Harbin, Heilongjiang 150025 (CN); KANG, Guifeng, Harbin, Heilongjiang 150025 (CN)
(74) Representative: M. Zardi & Co S.A.
(86) International application number: PCT/CN2024/081880
(87) International publication number: WO 2025/129826

(57) **Abstract**

Provided are an isoquinoline derivative, a synthesis method therefor, and use thereof, belonging to the technical field of biomedicine. Isoquinoline derivatives are a novel class of Pgk1 activators, which exhibit relatively high Pgk1 activation activity, demonstrating better efficacy than terazosin currently used in the art, and do not inhibit Pgk1. They have clinical potential for the prevention or treatment of neurodegenerative diseases and metabolic diseases.

## Description

### FIELD

The present disclosure belongs to the technical field of biomedicine, and more particularly, to an isoquinoline derivative, a synthesis method therefor, and use thereof.

### BACKGROUND

Decreased glucose metabolism in brain tissue is an important pathogenic factor in the occurrence of various neurodegenerative diseases.

The brain tissue consumes 20% to 25% of glucose in the human body, yet accounts for only about 2% of body weight, indicating that the brain tissue has an enormous energy demand and is a highly active organ in glucose metabolism. However, glucose metabolism is significantly decreased in the brain tissue of the elderly. Numerous studies have shown that decreased glucose metabolism in brain tissue occurs 10 years to 15 years earlier than the onset of clinical symptoms in patients with Alzheimer's disease (AD). Decreased glucose metabolism in brain tissue is a common pathological feature of various neurodegenerative diseases. Why is glucose metabolism so crucial for brain tissue? First, glucose metabolism is the primary pathway for cells to produce energy (ATP). As the most active cell type in the body, neurons require a large amount of ATP to maintain diverse physiological activities, such as protein synthesis, long-distance transport of proteins or organelles, generation of action potentials, release of neurotransmitters, pinocytosis, and exocytosis. Second, ATP plays a role in preventing non-specific protein aggregation and is known as a "hydrotrope". In the elderly, decreased glucose metabolism leads to a significant decrease in neuronal ATP concentration, which can be as low as 1 mM to 2 mM, while the normal ATP level in normal individual ranges from 8 mM to 10 mM. Low ATP concentrations induce pathological aggregation of various proteins, while high ATP concentrations can prevent non-specific protein aggregation. Therefore, decreased glucose metabolism is an important cause of brain aging. Improving glucose metabolism holds promise for improving brain health and preventing, alleviating, or even reversing brain aging. However, there are currently no internationally recognized clinical drugs that can improve glucose metabolism. Studies have found that resveratrol, metformin, rapamycin, vitamin B3, coenzyme Q10, creatine, insulin, etc., exert either no effect or clinical efficacy in only a small subset of the population. Currently, the most widely accepted methods for protecting the brain and muscles are exercise and dietary restriction. The mechanisms underlying the beneficial effects of exercise and dietary restriction mainly involve improving mitochondrial function and increasing glucose metabolism levels in various organs.

Decreased glucose metabolism in muscle tissue is an important factor contributing to the high prevalence of insulin resistance in the elderly.

The global prevalence of diabetes in adults is around 10%. Diabetes is an age-related disease. One study showed that the prevalence of diabetes was 21.1%, 47.0%, 66.7%, and 75.7% among people aged 20 to 39, 40 to 59, 60 to 74, and ≥75 years, respectively. Why are the elderly more prone to diabetes? It is well known that more than 80% of blood glucose in the human body is consumed by skeletal muscle. However, total muscle mass decreases by 8% per decade after age 40 and by 15% per decade after age 70, leading to a high prevalence of sarcopenia in the elderly. Even with normal insulin levels, patients with sarcopenia are unable to metabolize blood glucose through skeletal muscle, developing insulin resistance. Studies have found that for every 10% increase in the proportion of skeletal muscle in the human body, the risk of insulin resistance can decrease by 11%. In addition to sarcopenia, decreased glucose metabolism can be mediated by mitochondrial damage. A variety of factors can lead to mitochondrial damage, such as fatty acid accumulation, oxidative stress, gene mutations, and mitochondrial respiratory inhibitors, all of which can increase the risk of type II diabetes.

In fact, impaired glucose metabolism often occurs simultaneously in various organs of patients, including the brain and muscle tissue. Therefore, the neurodegenerative disease and insulin resistance often coexist in the same patient. Studies have found that 80% of patients with Alzheimer's disease and 58% of patients with Parkinson's disease are accompanied by diabetes or insulin resistance.

Quinazoline drugs can prevent and treat the neurodegenerative disease by targeting Pgk1 and activating glucose metabolism.

Quinazoline drugs are a class of α1-adrenergic receptor inhibitors used to treat benign prostatic hyperplasia or hypertension. Commonly used quinazoline drugs on the market include terazosin (TZ), alfuzosin, and dosazosin. Our team's previous studies found that terazosin can bind to phosphoglycerate kinase 1 (Pgk1). There are ten enzymes in the glycolysis, and Pgk1 is the seventh one, and also the first enzyme that generates ATP. Terazosin can activate Pgk1, which in turn activates the glycolysis. Pyruvate produced by glycolysis enters the mitochondrial tricarboxylic acid cycle (TCA cycle), leading to an increase in mitochondrial oxidative phosphorylation metabolism and a large amount of ATP production in the cell. The cascade of glycolysis and the mitochondrial TCA cycle constitutes the glucose metabolism pathway.

Studies have shown that terazosin exerts therapeutic and prophylactic effects in mouse and rat models of Parkinson's disease, Alzheimer's disease, multiple system atrophy, and other related diseases. These effects are supported by patient data. Analysis of multiple epidemiological studies has revealed that the incidence of Parkinson's disease and related diseases is significantly reduced in populations with long-term use of quinazoline drugs. In contrast, other types of α1-adrenergic receptor inhibitors, such as tamsulosin, do not exhibit similar effects.

Quinazoline drugs have safety concerns, so the modification of quinazoline drugs is of great research significance.

In principle, a drug that activates glucose metabolism should exert a hypoglycemic effect. The results of multiple clinical trials have shown that terazosin or doxazosin can indeed improve blood glucose and lipid levels in diabetic patients, but their efficacy is extremely limited or ineffective. In addition, no reports have shown that quinazoline drugs can reduce blood glucose in mice or rats. Combined administration of an α1-adrenergic receptor inhibitor (terazosin) and a β-adrenergic receptor inhibitor (propranolol) did not affect blood glucose in rats. These results indicate that quinazoline drugs exert weak or no effect in activating glucose metabolism in skeletal muscle.

Adrenergic receptor inhibitors such as terazosin, alfuzosin, doxazosin, and tamsulosin can produce various side effects, such as intraoperative floppy iris syndrome (IFIS), hypotension, fatigue, dizziness, syncope, and blurred vision, leading to treatment discontinuation in 10% to 20% of patients. Studies have found that the binding of terazosin to the α1 adrenergic receptor depends on a nitrogen atom on the quinazoline ring. Substitution of this nitrogen atom with a carbon atom can reduce the affinity for the α1 adrenergic receptor by more than 1000-fold. Current research shows that the modified terazosin (TZ-md) still retains the effect of activating Pgk1, but does not inhibit the α1 adrenergic receptor.

In addition, low concentrations of terazosin can activate Pgk1, while high concentrations of terazosin can inhibit Pgk1. The mechanism underlying this phenomenon is as follows: the binding site of terazosin on the Pgk1 protein overlaps with the binding sites of the substrate ADP and the product ATP. Since the affinity of terazosin for Pgk1 is more than 10-fold higher than that of ADP, high-dose terazosin binds more to the Pgk1 protein, thereby inhibiting the binding of the substrate ADP and consequently suppressing Pgk1 enzymatic activity. The rate-limiting step of the Pgk1 enzymatic reaction is the dissociation of ATP from the Pgk1 protein, which accounts for nearly 96% of the total enzymatic reaction time. The affinity of terazosin for Pgk1 is more than 100-fold higher than that of ATP. Therefore, low-dose of terazosin can competitively displace ATP binding, accelerate the Pgk1 enzymatic reaction, and activate Pgk1. A recent prospective clinical trial found that after treatment with terazosin, individual patients with Parkinson's disease exhibited a decrease in ATP levels in brain tissue and red blood cells, which is distinct from the increase in ATP levels observed in most patients. This result suggests that terazosin may exert an inhibitory effect on Pgk1 in these patients, which is more likely to occur in patients with a compromised blood-brain barrier. Meanwhile, existing studies indicate that 13.5% of patients in a population with an average age of 70 years exhibit blood-brain barrier disruption.

In summary, quinazoline drugs, as PgK1 activators, have three main weaknesses. 1) The quinazoline drugs are insufficient in activating glucose metabolism in skeletal muscle and lack hypoglycemic efficacy, suggesting that quinazoline drugs may not be the optimal compounds for activating PgK1. 2) The inhibition of α1 adrenergic receptors by quinazoline drugs is associated with a variety of adverse side effects, leading to treatment discontinuation in some patients. 3) Quinazoline drugs carry the risk of inhibiting PgK1, resulting in ineffective treatment of the related diseases.

### SUMMARY

Based on three shortcomings of the above-described quinazoline drugs, the present disclosure provides an isoquinoline derivative, a synthesis method therefor, and use thereof. The isoquinoline derivative, as a novel class of Pgk1 activators, exhibits high Pgk1 activation activity and has clinical potential for the prevention or treatment of neurodegenerative diseases and metabolic diseases.

To achieve the above-described objectives, the present disclosure provides the following technical solutions:

A first technical solution of the present disclosure provides an isoquinoline derivative represented by a general structural formula as follows: 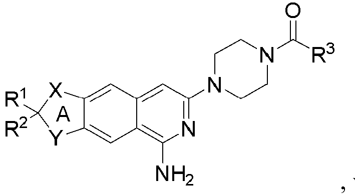 wherein: ring A represents an optionally substituted five-membered oxygen-containing heterocycle; X is O or C, Y is O or C, and at least one of X and Y is O; R¹ represents hydrogen or methyl; R² represents hydrogen or methyl; and R³ is five-membered cycloalkyl or five-membered heterocyclic group containing one heteroatom selected from an oxygen atom or a nitrogen atom. A structure of the isoquinoline derivative has any one of the following structures:

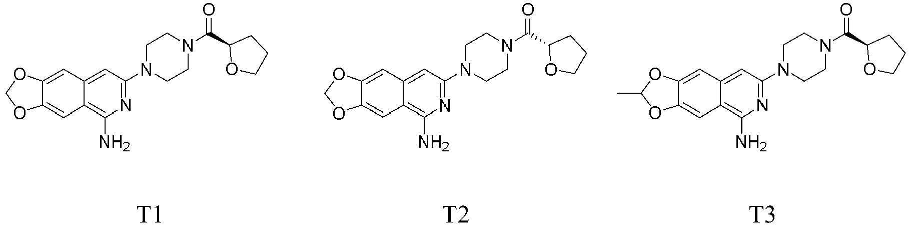

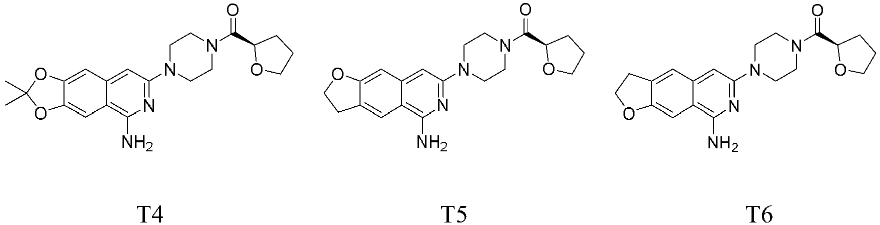

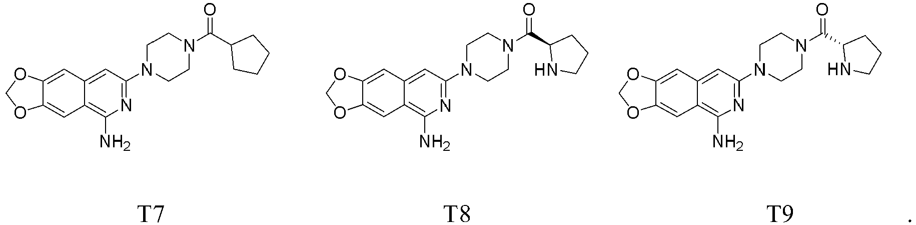

A second technical solution of the present disclosure provides a pharmaceutically acceptable salt of the above-described isoquinoline derivative.

Preferably, the pharmaceutically acceptable salt of the isoquinoline derivative includes hydrochloride, sulfate, hydrobromide, nitrate, trifluoroacetate, acetate, phosphate, benzenesulfonate, p-toluenesulfonate, methanesulfonate, oxalate, tartrate, fumarate, maleate, succinate, malate, benzoate, citrate, or lactate.

The present disclosure verifies that hydrochloride, sulfate, and trifluoroacetate thereof all exhibit the same efficacy as the isoquinoline derivative, and other pharmaceutically acceptable salts should also exhibit the same efficacy as the isoquinoline derivative.

A third technical solution of the present disclosure provides an isoquinoline derivative formulation. The formulation includes, as an active ingredient:the above-described isoquinoline derivative; and/or the above-described pharmaceutically acceptable salt of the isoquinoline derivative.

Preferably, the formulation is respective pharmaceutical formulations prepared from the isoquinoline derivative or pharmaceutically acceptable salt thereof with a pharmaceutically acceptable excipient, such as water, alcohol, vegetable oil, starch, microcrystalline cellulose, lactose, gelatin, magnesium stearate, sodium alginate, maltodextrin, talc, crospovidone, gum, petrolatum, and the like.

A fourth technical solution of the present disclosure provides a phosphoglycerate kinase activator. The activator is the above-described isoquinoline derivative, the above-described pharmaceutically acceptable salt of the isoquinoline derivative, or the above-described isoquinoline derivative formulation.

A fifth technical solution of the present disclosure provides a medicament for treating or preventing a neurodegenerative disease. The medicament is the above-described isoquinoline derivative, the above-described pharmaceutically acceptable salt of the isoquinoline derivative, or the above-described isoquinoline derivative formulation.

Preferably, the neurodegenerative disease includes, but is not limited to, Alzheimer's disease, Parkinson's disease, amyotrophic lateral sclerosis (ALS), Huntington's disease, etc.

A sixth technical solution of the present disclosure provides a medicament for treating or preventing a metabolic disease. The medicament is the above-described isoquinoline derivative, the above-described pharmaceutically acceptable salt of the isoquinoline derivative, or the above-described isoquinoline derivative formulation.

Preferably, the metabolic disease includes, but is not limited to, diabetes, obesity, etc.

A seventh technical solution of the present disclosure provides a method for preparing the above-described isoquinoline derivative. The method includes one of the following synthetic routes:

### Synthetic route A:

Adding malonic acid and piperidine to a solution of compound I for reaction to obtain compound II; reacting compound II in the presence of a catalyst under a H₂ atmosphere to obtain compound III; reacting compound III with an oxalyl chloride to generate an acyl chloride, then reacting under catalysis of aluminum trichloride to obtain compound IV; reacting compound IV with isoamyl nitrite under an acidic condition to obtain compound V; reacting compound V with phosphorus oxychloride to obtain compound VI; reacting compound VI with an amino protecting reagent in N-methylpyrrolidone to obtain compound VII; reacting compound VII, compound XI, dioxane, Pd₂(dba)₃, RuPhos, and cesium carbonate together to obtain compound VIII; deprotecting compound VIII to remove a Boc protecting group to obtain compound IX; reacting compound IX with R³-COOH to obtain compound X; and reacting compound X with trifluoroacetic acid to remove an amino protecting group to obtain isoquinoline derivative compound T, the reaction route A being as follows:

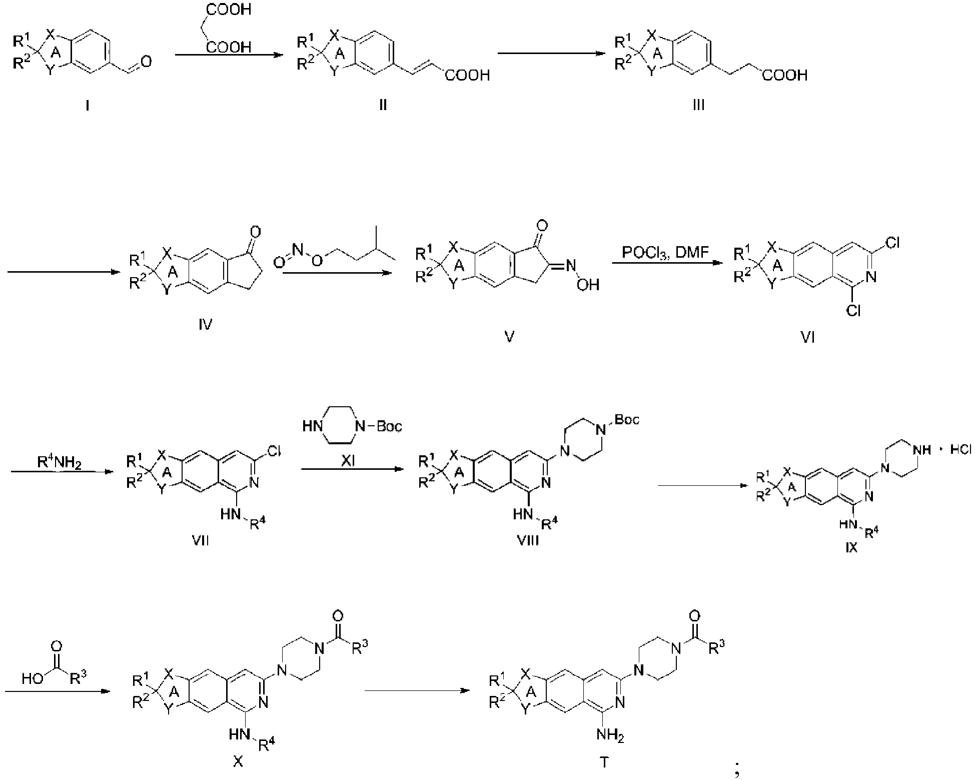

Preferably, the reaction temperature and reaction duration for each step in synthetic route A are not particularly limited, provided that the starting materials can be converted to the target product.

### Synthetic route B:

Reacting compound XI with R³-COOH, O-(7-azabenzotriazol-1-yl)-N,N,N',N'-tetramethyluronium hexafluorophosphate, and N,N-diisopropylethylamine in a solvent to obtain compound XII; deprotecting compound XII to remove a Boc protecting group to obtain compound XIII; reacting compound XIII with compound VII obtained according to synthetic route A, dioxane, Pd₂(dba)₃, RuPhos, and cesium carbonate together to obtain compound X; and reacting compound X with trifluoroacetic acid to remove an amino protecting group to obtain isoquinoline derivative compound T, the reaction route B being as follows:

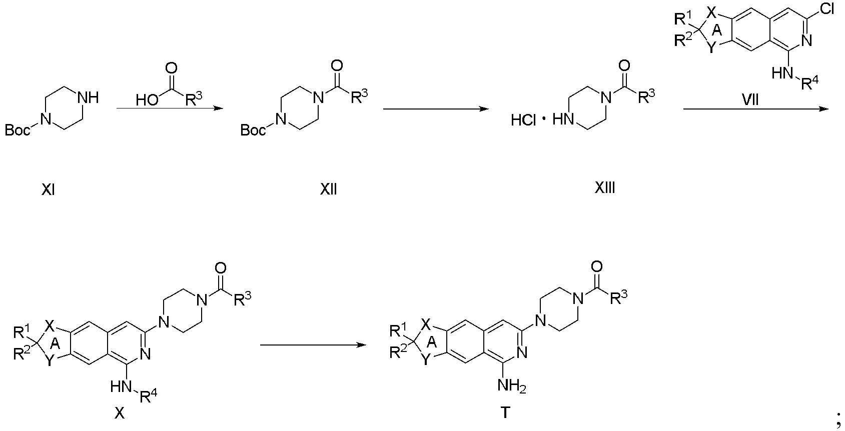

Preferably, the reaction temperature and reaction duration for each step in synthetic route B are not particularly limited, provided that the starting materials can be converted to the target product; and

### Synthetic route C:

Reacting compound VI obtained according to synthetic route A with ammonia water to obtain compound XIV; reacting compound XIV with an amino protecting reagent to obtain compound VII; and treating compound VII according to a method of synthetic route A to obtain isoquinoline derivative Compound T, the reaction route C being as follows:

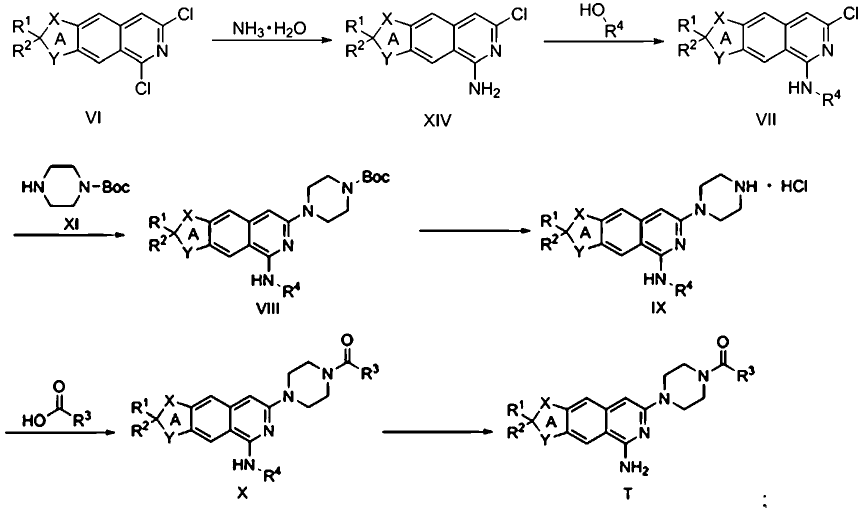

Preferably, the reaction temperature and reaction duration for each step in synthetic route C are not particularly limited, provided that the starting materials can be converted to the target product. R⁴ of compounds involved in synthetic route A, synthetic route B, and synthetic route C is the amino protecting group.

The beneficial technical effects of the present disclosure are as follows:

The isoquinoline derivative and salt thereof synthesized in the present disclosure have been verified to activate Pgk1 by targeting Pgk1, achieving a purpose of treating or preventing the neurodegenerative diseases and the metabolic diseases. The isoquinoline derivative and salt thereof exhibit superior efficacy compared to terazosin and do not cause the inhibition of Pgk1.

### BRIEF DESCRIPTION OF THE DRAWINGS

FIG. 1 shows effects of various isoquinoline derivatives prepared in examples of the present disclosure and terazosin on cellular ATP activity.
FIG. 2 shows results of *in vitro* biochemical assays of drugs on Pgk1 enzymatic activity, in which, A represents a positive control, showing effects of different concentrations of terazosin on Pgk1 enzymatic activity; and B represents an experimental group, showing effects of different concentrations of T6 on Pgk1 enzymatic activity.
FIG. 3 shows effects of different concentrations of compound T6 and terazosin on Pgk1 enzymatic activity in cells, in which, A represents an effect of terazosin on Pgk1 enzymatic activity in cells, and B represents an effect of compound T6 on Pgk1 enzymatic activity in cells.
FIG. 4 shows effects of different concentrations of compounds T6 and terazosin on ATP levels in cells.
FIG. 5 shows effects of various isoquinoline derivatives prepared in examples of the present disclosure and terazosin on blood glucose in mice.
FIG. 6 shows effects of liraglutide and different concentrations of compound T6 on blood glucose in diabetic model mice.
FIG. 7 shows effects of metformin, terazosin, and compound T6 on blood glycated hemoglobin, insulin levels, and body weight in diabetic model mice, in which, A represents glycated hemoglobin levels, B represents insulin levels, and C represents changes in body weight.
FIG. 8 shows effects of terazosin and different concentrations of compound T2 on a motor ability and tyrosine hydroxylase levels in MPTP-induced Parkinson's disease model mice, in which, A represents an effect of each drug on the motor ability of MPTP-induced Parkinson's disease model mice, B represents an effect of terazosin on the tyrosine hydroxylase levels in MPTP-induced Parkinson's disease model mice, and C represents an effect of compound T2 at a dose of 0.1 mg/Kg on the tyrosine hydroxylase levels in MPTP-induced Parkinson's disease model mice.

### DETAILED DESCRIPTION

Various exemplary embodiments of the present disclosure are now described in detail. This detailed description should not be considered as a limitation of the present disclosure, but rather as a more detailed description of certain aspects, features, and embodiments of the present disclosure. It should be understood that the terminology used in the present disclosure is merely for describing particular embodiments and is not intended to limit the present disclosure.

In addition, regarding numerical ranges in the present disclosure, it should be understood that each intermediate value between the upper and lower limits of the range is also specifically disclosed. Every smaller range between any stated value or intermediate value within a stated range, and any other stated value or intermediate value within the range, is also encompassed within the present disclosure. The upper and lower limits of these smaller ranges may be independently included or excluded from the range.

Unless otherwise stated, all technical and scientific terms used herein have the same meaning as commonly understood by one of ordinary skill in the art. Although only preferred methods and materials are described herein, any methods and materials similar or equivalent to those described herein can also be used in the implementation or testing of the present disclosure.

The terms "comprising," "including," "having," "containing," etc., as used herein are open-ended terms, meaning "including but not limited to".

### Example 1: Synthesis of Compound T1-II

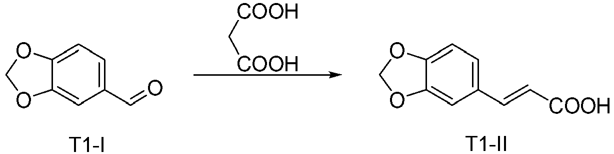

35 mL of DMF was added to a 250 mL eggplant-shaped flask. Under stirring, 9.01 g (60 mmol) of piperonal and 8.74 g (84.0 mmol) of malonic acid were added, followed by 0.66 g (7.8 mmol) of piperidine. The obtained light brown solution was stirred in an oil bath at 90°C for 10 hours. After cooling to room temperature, 60 mL of purified water was added. The mixture was stirred for 1 hour and then filtered. The filter cake was washed with purified water, and dried under vacuum at room temperature to obtain 8.67 g of the light brown intermediate T1-II, with a yield of 75.2%. NMR analysis was as follows:

¹H NMR: (300 MHz, DMSO-d₆) δ 12.24 (brs, 1H), 7.52 (d, J = 15.9 Hz, 1H), 7.37 (d, J = 1.6 Hz, 1H), 7.16 (dd, J1 = 8.1 Hz, J2 = 1.6 Hz, 1H), 6.95 (d, J = 8.1 Hz, 1H), 6.40 (d, J = 15.9 Hz, 1H), 6.08 (s, 2H).

MS (C₁₀H₈O₄): 191.67 [M-H]⁻, 383.85 [2M-H]⁻.

### Example 2: Synthesis of Compound T1-III

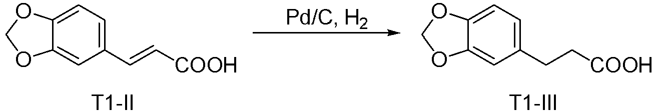

250 mL of tetrahydrofuran was added to a 500 mL eggplant-shaped flask, and 4.19 g (2.18 mmol) of intermediate T1-II and 0.84 g of palladium on carbon were added under stirring. The obtained mixture was stirred at room temperature under a hydrogen atmosphere for 20 hours. The reaction mixture was filtered through diatomaceous earth, the filtrate was concentrated under reduced pressure, and the residue was recrystallized from ethyl acetate/petroleum ether to obtain 2.97 g of white intermediate T1-III, with a yield of 70.1%. NMR analysis was as follows:

¹H NMR: (300 MHz, CDCl3) δ 11.81 (brs, 1H), 6.74 (d, J = 7.9 Hz, 1H), 6.70 (d, J = 1.4 Hz, 1H), 6.66 (dd, J1 = 7.9 Hz, J2 = 1.7 Hz, 1H), 5.93 (s, 2H), 2.88 (t, J = 7.8 Hz, 2H), 2.64 (t, J = 7.8 Hz, 2H).

MS (C₁₀H₁₀O₄): 193.57 [M-H]⁻, 387.79 [2M-H]⁻.

### Example 3: Synthesis of Compound T1-IV

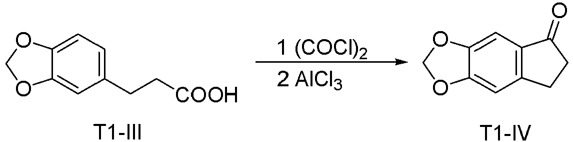

50 mL of dichloromethane was added to a 250 mL eggplant-shaped flask, and 4.40 g (22.66 mmol) of intermediate T1-III and 0.17 mL of DMF were added under stirring. The obtained light brown solution was stirred in an ice bath. 5.75 g (45.32 mmol) of oxalyl chloride was added dropwise to the above-described reaction solution under stirring. The reaction solution was gradually warmed to room temperature and stirred overnight (approximately 12 hours). The reaction solution was concentrated by rotary evaporation under vacuum to obtain a brownish-yellow oil. An additional 80 mL of dichloromethane was added in an ice bath, followed by addition of 4.53 g (33.99 mmol) of aluminum trichloride in three equal portions. The obtained reddish-brown mixture was stirred in an ice bath for 3 hours. 80 g of ice water was added (in three equal portions), and the mixture was stirred for 0.5 hour and separated. The aqueous phase was extracted twice with dichloromethane (40 mL) (the volumes in parentheses represented the amount of solution used for each extraction or washing, and had the same meaning in the following examples). The organic phase was combined and washed successively three times with saturated sodium bicarbonate (100 mL), once with saturated sodium chloride (50 mL), dried over anhydrous sodium sulfate. The solvent was removed by rotary evaporation under vacuum, and the obtained residue was purified by column chromatography (dichloromethane:methanol = 200:1-150:1-100:1) to obtain 2.37 g of intermediate T1-IV, with a yield of 59.4%. NMR analysis was as follows:

¹H NMR: (300 MHz, CDCl3) δ 7.03 (s, 1H), 6.79 (s, 1H), 6.02 (s, 2H), 2.97 (m, 2H), 2.62 (m, 2H).

MS (C₁₀H₈O₃): 176.96 [M+H]⁺.

### Example 4: Synthesis of Compound T1-V

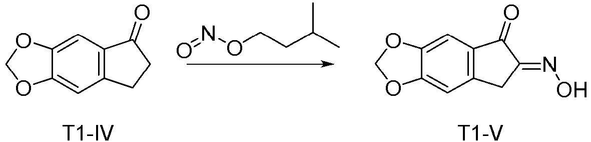

65 mL of methanol was added to a 100 mL eggplant-shaped flask, and 6.52 g (37.01 mmol) of intermediate T1-IV and 1.08 mL (12.95 mmol) of concentrated hydrochloric acid were added under stirring. 6.50 g (55.51 mmol) of isoamyl nitrite was added dropwise to the obtained nearly colorless solution, and the mixture was stirred at room temperature for 8 hours. The obtained reaction mixture was filtered, and the filter cake was washed with methanol (15 mL) and dried under vacuum at room temperature to obtain 7.43 g of yellow intermediate T1-V, with a yield of 97.8%. NMR analysis was as follows:

¹H NMR: (300 MHz, DMSO-d₆) δ 12.44 (s, 1H), 7.11 (s, 1H), 7.09 (s, 1H), 6.17 (s, 2H), 3.62 (s, 2H).

MS (C₁₀H₇NO₄): 223.11 [M+H₂O]⁺, 411.17 [2M+H]⁺, 433.11 [2M+Na]⁺.

### Example 5: Synthesis of Compound T1-VI

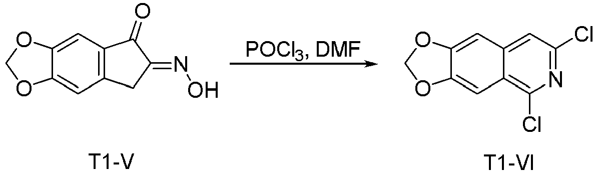

7.43 g of intermediate T1-V was added to a 250 mL eggplant-shaped flask, and then 75 mL of phosphorus oxychloride and 0.75 mL of DMF were added. The obtained yellow mixture was stirred and reacted in an oil bath at 100°C for 4 hours. After the completion of the reaction, phosphorus oxychloride was removed by rotary evaporation under vacuum. The residue was purified by column chromatography (dichloromethane:methanol = 400:1) to obtain 6.47 g of intermediate T1-VI, with a yield of 73.8%. NMR analysis was as follows:

¹H NMR: (300 MHz, DMSO-d₆) δ 7.86 (s, 1H), 7.48 (s, 1H), 7.41 (s, 1H), 6.30 (s, 2H).

MS (C₁₀H₅Cl₂NO₂): 241.99 [M+H]⁺.

### Example 6: Synthesis of Compound T1-VII

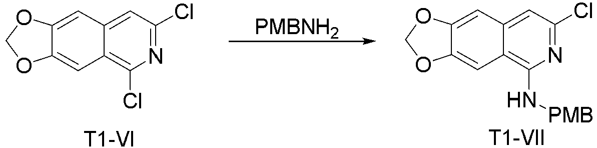

4.63 g (19.12 mmol) of intermediate T1-VI, 7.87 g (57.35 mmol) of p-methoxybenzylamine, and 46 mL of N-methylpyrrolidone were added to a 250 mL reaction tube. The obtained reaction mixture was reacted in an oil bath at 120°C under a nitrogen atmosphere for 3 hours. After the completion of the reaction, 150 mL of water and 150 mL of ethyl acetate were added, and the organic phase was collected. The aqueous phase was extracted twice with ethyl acetate (50 mL), and the combined organic phase was washed once with saturated sodium chloride (50 mL) and dried over anhydrous sodium sulfate. The solvent was removed by rotary evaporation under vacuum, and the obtained residue was purified by column chromatography (ethyl acetate:petroleum ether = 1:20-1:10-1:5-1:3-1:2) to obtain 5.51 g of intermediate T1-VII, with a yield of 84.0%. NMR analysis was as follows:

¹H NMR: (300 MHz, DMSO-d₆) δ 7.87 (t, J = 5.7 Hz, 1H), 7.73 (s, 1H), 7.31 (brs, 1H), 7.28 (brs, 1H), 7.11 (s, 1H), 6.89-6.88 (m, 1H), 6.86-6.85 (m, 2H), 6.15 (s, 2H), 4.58 (d, J = 5.7 Hz, 2H), 3.71 (s, 3H).

MS (C₁₈H₁₅ClN₂O₃): 343.09 [M+H]⁺.

### Example 7: Synthesis of Compound T1-VIII

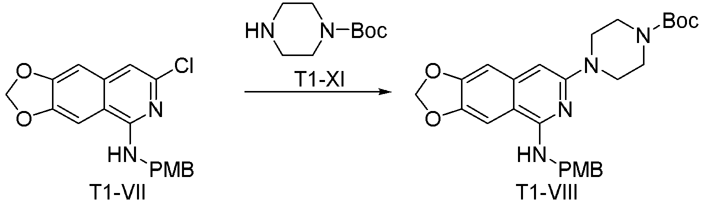

Dioxane (50 mL), 3.43 g (10.00 mmol) of intermediate T1-VII, 3.73 g (20.00 mmol) of N-Boc-piperazine, 0.73 g (0.80 mmol) of Pd₂(dba)₃, 0.56 g (1.2 mmol) of RuPhos, and 6.52 g (20.00 mmol) of cesium carbonate were added to a 200 mL reaction tube. The obtained reaction mixture was stirred and reacted at 100°C under a nitrogen atmosphere for 12 hours. After the completion of the reaction, the mixture was cooled to room temperature. The reaction mixture was filtered through diatomaceous earth, and 150 mL of water and 150 mL of ethyl acetate were added to the filtrate. The organic phase was collected, and the aqueous phase was extracted twice with ethyl acetate (50 mL). The combined organic phase was washed once with saturated sodium chloride (50 mL) and dried over anhydrous sodium sulfate. The solvent was removed by rotary evaporation under vacuum, and the obtained residue was purified by column chromatography (ethyl acetate:petroleum ether = 1:40-1:20-1:10-1:5-1:3) to obtain 3.46 g of intermediate T1-VIII,with a yield of 70.2%. NMR analysis was as follows:

¹H NMR: (300 MHz, CDCl3) δ 7.35-7.33 (m, 1H), 7.32-7.30 (m, 1H), 6.90-6.89 (m, 2H), 6.87-6.85 (m, 1H), 6.81 (s, 1H), 6.06 (s, 1H), 5.96 (s, 2H), 4.98 (m, 1H), 4.68-4.66 (m, 2H), 3.81 (s, 3H), 3.58-3.53 (m, 4H), 3.46-3.43 (m, 4H), 1.49 (s, 9H).

MS (C₂₇H₃₂N₄O₅): 493.28 [M+H]⁺.

### Example 8: Synthesis of Compound T1-IX

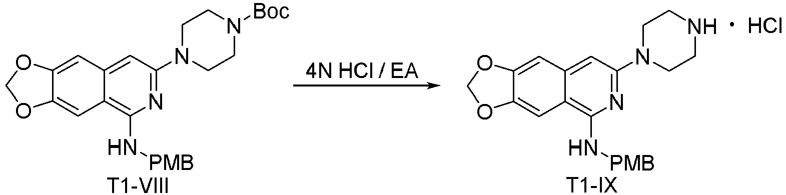

1.38 g (2.80 mmol) of intermediate T1-VIII and 27 mL of 4N hydrochloric acid in ethyl acetate were added to a 100 mL eggplant-shaped flask, and the obtained reaction mixture was stirred at room temperature for 7 hours. The solvent was removed by rotary evaporation under vacuum, and the obtained residue was washed with ethyl acetate (10 mL), filtered, and dried under vacuum at room temperature to obtain 1.10 g of intermediate T1-IX. The obtained product was used directly for the next reaction without further purification.

MS (C₂₂H₂₅ClN₄O₃): 393.32 [M+H-HCl]⁺.

### Example 9: Synthesis of Compound T1-X

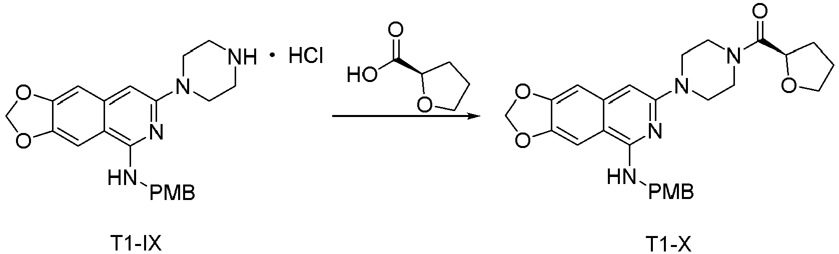

536 mg (1.25 mmol) of intermediate T1-IX, 175 mg (1.5 mmol) of (R)-(+)-tetrahydrofuran-2-carboxylic acid, 713 mg (1.875 mmol) of HATU, 485 mg (3.75 mmol) of DIEA, and 10 mL of DMF were added to a 50 mL eggplant-shaped flask. The obtained reaction mixture was stirred overnight at room temperature under a nitrogen atmosphere. Water (100 mL) and ethyl acetate (100 mL) were added, and the organic phase was collected. The aqueous phase was extracted twice with ethyl acetate (30 mL), and the combined organic phase was washed once with saturated sodium chloride (30 mL) and dried over anhydrous sodium sulfate. The mixture was concentrated under reduced pressure, and the obtained residue was purified by column chromatography (ethyl acetate:petroleum ether = 1:20-1:10-1:5-1:3-1:1-2:1-3:1) to obtain 324 mg of intermediate T1-X, with a yield of 52.9%. NMR analysis was as follows:

¹H NMR: (300 MHz, CDCl3) δ 7.34-7.33 (m, 1H), 7.31 (m 1H), 6.89-6.85 (m, 3H), 6.82 (s, 1H), 6.06 (s, 1H), 5.97 (s, 2H), 5.01 (brs, 1H), 4.67 (m, 3H), 3.98-3.86 (m, 3H), 3.81 (s, 3H), 3.76-3.67 (m, 3H), 3.53-3.47 (m, 4H), 2.32-2.28 (m, 1H), 2.08-1.92 (m, 3H).

MS (C₂₇H₃₀N₄O₅): 491.28 [M+H]⁺.

### Example 10: Synthesis of Compound T1

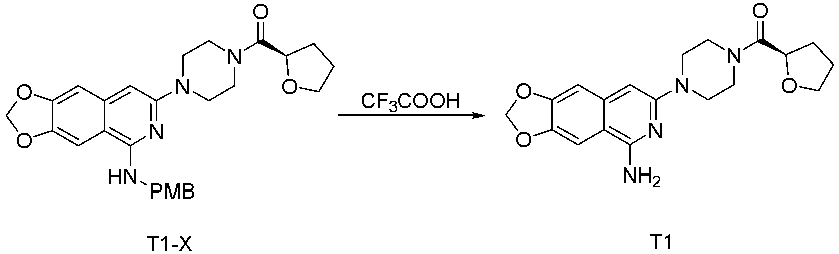

200 mg (0.41 mmol) of intermediate T1-X and 2 mL of trifluoroacetic acid were added to a 4 mL reaction tube. The obtained reaction mixture was stirred in an oil bath at 45°C for 3 hours, and the solvent was removed by rotary evaporation under vacuum. 15 mL of saturated NaHCO₃ solution was added to the residue, and the mixture was extracted three times with dichloromethane (15 mL). The organic phase was combined, washed once with saturated sodium chloride (15 mL), and dried over anhydrous sodium sulfate. The mixture was concentrated under reduced pressure, and the obtained residue was purified by column chromatography (dichloromethane:methanol = 100:1-80:1-50:1-20:1-10:1) to obtain 62 mg of compound T1, with a yield of 41.1%. NMR analysis was as follows:

¹H NMR: (300 MHz, DMSO-d₆) δ 7.60 (s, 1H), 7.00 (s, 1H), 6.55 (brs, 2H), 6.25 (s, 1H), 6.10 (s, 2H), 4.68 (m, 1H), 3.81-3.70 (m, 4H), 3.65-3.45 (m, 6H), 2.09-1.98 (m, 2H), 1.88-1.79 (m, 2H).

MS (C₁₉H₂₂N₄O₄): 371.05 [M+H]⁺.

### Example 11: Synthesis of Compound T1-XII

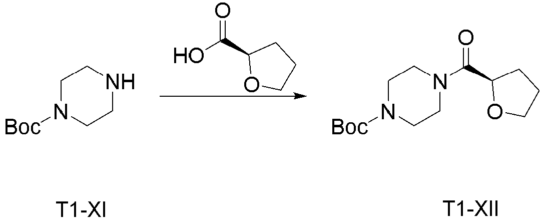

1.16 g (10.0 mmol) of (R)-(+)-tetrahydrofuran-2-carboxylic acid and 20 mL of DMF were added to a 100 mL reaction tube. 1.86 g (10.0 mmol) of intermediate T1-XI, 4.56 g (12.0 mmol) of HATU, and 2.59 g (20.0 mmol) of N,N-diisopropylethylamine were added to the obtained reaction solution. The obtained reaction solution was stirred at room temperature for 12 hours. After the completion of the reaction, 100 mL of water and 100 mL of ethyl acetate were added, and the organic phase was collected. The aqueous phase was extracted twice with ethyl acetate (50 mL), and the combined organic phase was washed once with saturated sodium chloride (50 mL) and dried over anhydrous sodium sulfate. The solvent was removed by rotary evaporation under vacuum, and the obtained residue was purified by column chromatography (ethyl acetate:petroleum ether = 1:20-1:10-1:7-1:5-1:2) to obtain 1.95 g of intermediate T1-XII, with a yield of 68.7%. The NMR analysis was as follows:

¹H NMR: (300 MHz, DMSO-d₆) δ 4.56-4.52 (m, 1H), 3.90-3.75 (m, 2H), 3.63-3.59 (m, 2H), 3.44-3.28 (m, 6H), 2.32-2.21 (m, 1H), 1.99-1.87 (m, 3H), 1.41 (s, 9H).

MS (C₁₄H₂₄N₂O₄): 285.14 [M+H]⁺.

### Example 12: Synthesis of Compound T1-XIII

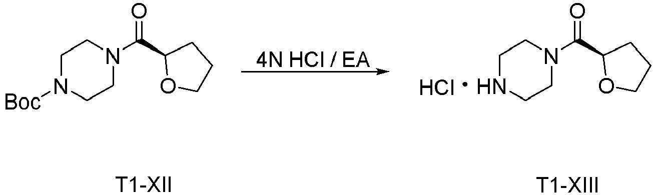

1.42 g (5.0 mmol) of intermediate T1-XII and 28 mL of 4N hydrochloric acid in ethyl acetate were added to a 100 mL eggplant-shaped flask, and the obtained reaction mixture was stirred at room temperature for 5 hours. The solvent was removed by rotary evaporation under vacuum, and the obtained residue was washed with ethyl acetate (10 mL), filtered, and dried under vacuum at room temperature to obtain 0.95 g of intermediate T1-XIII. The obtained product was used directly for the next reaction without further purification.

MS (C₉H₁₇ClN₂O₂): 185.17 [M+H-HCl]⁺.

### Example 13: Synthesis of Compound T1-X

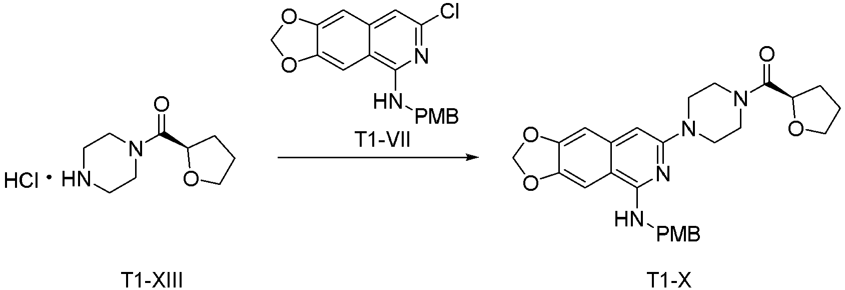

40 mL of dioxane, 2.58 g (7.50 mmol) of intermediate T1-VII, 3.3 g (15.00 mmol) of intermediate T1-XIII, 0.55 g (0.60 mmol) of Pd₂(dba)₃, 0.42 g (0.90 mmol) of RuPhos, and 4.89 g (15.00 mmol) of cesium carbonate were added to a 200 mL reaction tube. The obtained reaction mixture was stirred at 100°C under a nitrogen atmosphere for 12 hours. After the completion of the reaction, the mixture was cooled to room temperature. The reaction mixture was filtered through diatomaceous earth, and 120 mL of water and 120 mL of ethyl acetate were added to the filtrate. The organic phase was collected, and the aqueous phase was extracted twice with ethyl acetate (50 mL). The combined organic phase was washed once with saturated sodium chloride (50 mL) and dried over anhydrous sodium sulfate. The solvent was removed by rotary evaporation under vacuum, and the obtained residue was purified by column chromatography (ethyl acetate:petroleum ether = 1:20-1:10-1:5-1:3-1:1-2:1-3:1) to obtain 1.85 g of intermediate T1-X, with a yield of 50.1%.

### Example 14: Synthesis of Compound T1-XIV

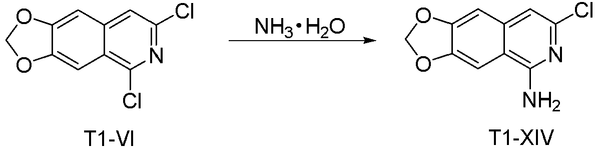

1.09 g (4.50 mmol) of intermediate T1-VI, 30 mL of dioxane, and 30 mL of ammonia were added to a 100 mL hydrothermal reactor. The obtained reaction mixture was stirred at 150°C for 48 hours. After the completion of the reaction, the mixture was cooled to room temperature. 30 mL of water and 30 mL of dichloromethane were added to the reaction mixture, and the organic phase was collected. The aqueous phase was extracted twice with dichloromethane (15 mL), and the combined organic phase was washed once with saturated sodium chloride (30 mL) and dried over anhydrous sodium sulfate. The solvent was removed by rotary evaporation under vacuum. The obtained residue was slurried twice with ethyl acetate (20 mL) at room temperature to obtain 0.64 g of intermediate T1-XIV, with a yield of 64.0%. The ¹H NMR analysis was as follows:

¹H NMR: (300 MHz, DMSO-d₆) δ 7.61 (s, 1H), 7.10 (s, 1H), 6.94 (brs, 2H), 6.84 (s, 1H), 6.14 (s, 2H).

MS (C₁₀H₇ClN₂O₂): 223.08 [M+H]⁺.

### Example 15: Synthesis of Compound T1-VII

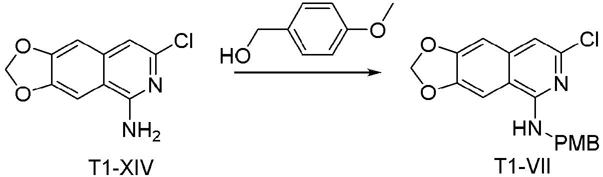

520 mg (2.34 mmol) of intermediate T1-XIV, 79 mg (0.35 mmol) of palladium acetate, 256 mg (0.70 mmol) of sodium 3-(diphenylphosphino)benzenesulfonate, 1.29 g (9.36 mmol) of p-methoxybenzyl alcohol, and 15 mL of water were added to a 100 mL hydrothermal reactor. The obtained reaction mixture was stirred at 140°C for 24 hours. Afterthe completion of the reaction, the reaction mixture was filtered through diatomaceous earth. 30 mL of water and 30 mL of ethyl acetate were added, and the organic phase was collected. The aqueous phase was extracted twice with ethyl acetate (15 mL), and the combined organic phase was washed once with saturated sodium chloride (30 mL) and dried over anhydrous sodium sulfate. The solvent was removed by rotary evaporation under vacuum, and the obtained residue was purified by column chromatography (ethyl acetate:petroleum ether = 1:20-1:10-1:7-1:5-1:2) to obtain 592 mg of intermediate T1-VII, with a yield of 74.0%.

### Example 16: Synthesis of Compound T2

The synthesis of the starting material T2-X was performed by reference to the synthetic method for T1-X.

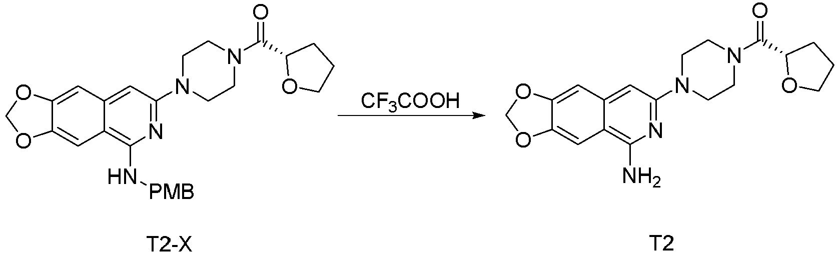

180 mg (0.37 mmol) of intermediate T2-X and 1.8 mL of trifluoroacetic acid were added to a 4 mL reaction tube. The obtained reaction mixture was stirred in an oil bath at 45°C for 3 hours, and the solvent was removed by rotary evaporation under vacuum. 15 mL of saturated NaHCO₃ solution was added to the obtained residue, and the mixture was extracted three times with dichloromethane (15 mL). The organic phase was combined, washed once with saturated sodium chloride (15 mL), and dried over anhydrous sodium sulfate. The mixture was concentrated under reduced pressure, and the obtained residue was purified by column chromatography (dichloromethane:methanol = 100:1-80:1-50:1-20:1-10:1) to obtain 65 mg of compound T2, with a yield of 47.8%. NMR analysis was as follows:

¹H NMR: (300 MHz, DMSO-d₆) δ 7.46 (s, 1H), 6.89 (s, 1H), 6.44 (brs, 2H), 6.10 (s, 1H), 6.04 (s, 2H), 4.71 (m, 1H), 3.82-3.70 (m, 4H), 3.66-3.56 (m, 6H), 2.07-1.99 (m, 2H), 1.88-1.80 (m, 2H).

MS (C₁₉H₂₂N₄O₄): 371.20 [M+H]⁺.

### Example 17: Synthesis of Compound T3

The synthesis of the starting material T3-X was performed by reference to the synthetic method for T1-X.

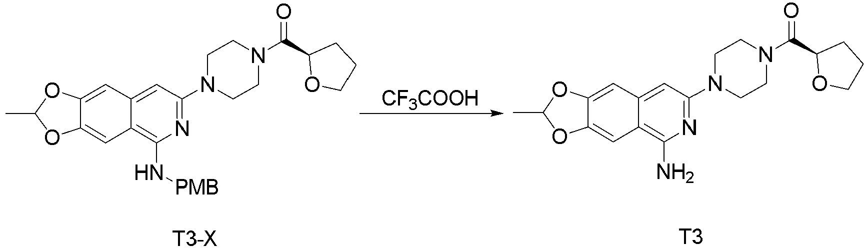

200 mg (0.37 mmol) of intermediate T3-X and 2.0 mL of trifluoroacetic acid were added to a 4 mL reaction tube. The obtained reaction mixture was stirred in an oil bath at 45°C for 3 hours, and the solvent was removed by rotary evaporation under vacuum. 15 mL of saturated NaHCO₃ solution was added to the obtained residue, and the mixture was extracted three times with dichloromethane (15 mL). The organic phase was combined, washed once with saturated sodium chloride (15 mL), and dried over anhydrous sodium sulfate. The mixture was concentrated under reduced pressure, and the obtained residue was purified by column chromatography (dichloromethane:methanol = 100:1-80:1-50:1-20:1-15:1) to obtain 78 mg of compound T3, with a yield of 51.3%. NMR analysis was as follows:

¹H NMR: (300 MHz, DMSO-d₆) δ 7.42 (s, 1H), 6.86 (s, 1H), 6.52 (brs, 2H), 6.30 (q, J = 5.0 Hz, 1H), 6.19(s, 1H), 4.75 (m, 1H), 3.85-3.68 (m, 4H), 3.72-3.60 (m, 6H), 2.05-1.98 (m, 2H), 1.90-1.80 (m, 2H), 1.67 (d, J = 5.0 Hz, 3H).

MS (C₂₀H₂₄N₄O₄): 385.23 [M+H]⁺.

### Example 18: Synthesis of Compound T4

The synthesis of the starting material T4-X was performed by reference to the synthetic method forT1-X.

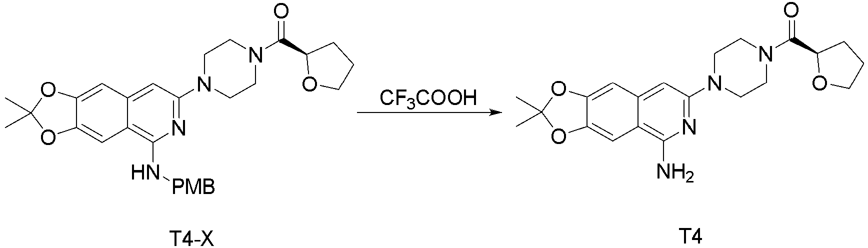

182 mg (0.35 mmol) of intermediate T4-X and 1.9 mL of trifluoroacetic acid were added to a 4 mL reaction tube. The obtained reaction mixture was stirred in an oil bath at 45°C for 3 hours, and the solvent was removed by rotary evaporation under vacuum. 15 mL of saturated NaHCO₃ solution was added to the obtained residue, and the mixture was extracted three times with dichloromethane (15 mL). The organic phase was combined, washed once with saturated sodium chloride (15 mL), and dried over anhydrous sodium sulfate. The mixture was concentrated under reduced pressure, and the obtained residue was purified by column chromatography (dichloromethane:methanol = 100:1-80:1-50:1-20:1-15:1) to obtain 78 mg of compound T4, with a yield of 55.7%. NMR analysis was as follows:

¹H NMR: (300 MHz, DMSO-d₆) δ 7.53 (s, 1H), 6.95 (s, 1H), 6.56 (brs, 2H), 6.15 (s, 1H), 4.76 (m, 1H), 3.80-3.66 (m, 4H), 3.70-3.55 (m, 6H), 2.10-1.99 (m, 2H), 1.86-1.78 (m, 2H), 1.72 (s, 6H).

MS (C₂₁H₂₆N₄O₄): 399.25 [M+H]⁺.

### Example 19: Synthesis of Compound T5

The synthesis of the starting material T5-X was performed by reference to the synthetic method for T1-X.

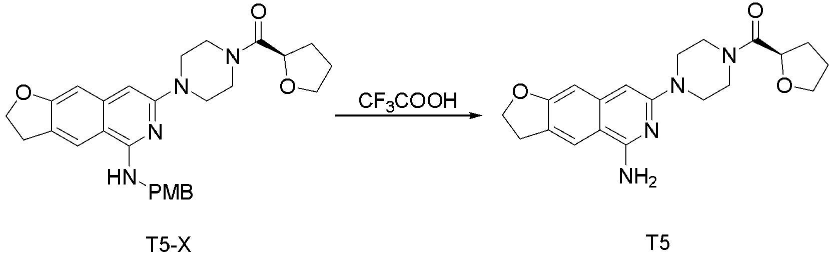

245 mg (0.50 mmol) of intermediate T5-X and 2.5 mL of trifluoroacetic acid were added to a 4 mL reaction tube. The obtained reaction mixture was stirred in an oil bath at 45°C for 3 hours, and the solvent was removed by rotary evaporation under vacuum. 15 mL of saturated NaHCO₃ solution was added to the obtained residue, and the mixture was extracted three times with dichloromethane (15 mL). The organic phase was combined, washed once with saturated sodium chloride (15 mL), and dried over anhydrous sodium sulfate. The mixture was concentrated under reduced pressure, and the obtained residue was purified by column chromatography (dichloromethane:methanol = 100:1-80:1-50:1-20:1-15:1) to obtain 85 mg of compound T5, with a yield of 45.9%. NMR analysis was as follows:

¹H NMR: (300 MHz, DMSO-d₆) δ 7.98 (s, 1H), 7.17 (s, 1H), 6.50 (s, 2H), 6.29 (s, 1H), 4.62 (t, J = 8.6 Hz, 2H), 3.26 (t, J = 8.6 Hz, 2H), 4.72 (m, 1H), 3.84-3.71 (m, 4H), 3.66-3.48 (m, 6H), 2.10-2.00 (m, 2H), 1.90-1.80 (m, 2H).

MS (C₂₀H₂₄N₄O₃): 369.15 [M+H]⁺.

### Example 20: Synthesis of Compound T6

The synthesis of the starting material T6-X was performed by reference to the synthetic method for T1-X.

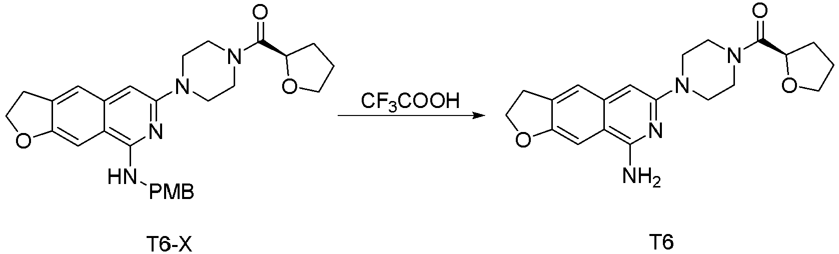

220 mg (0.45 mmol) of intermediate T6-X and 2.2 mL of trifluoroacetic acid were added to a 4 mL reaction tube. The obtained reaction mixture was stirred in an oil bath at 45°C for 3 hours, and the solvent was removed by rotary evaporation under vacuum. 15 mL of saturated NaHCO₃ solution was added to the obtained residue, and the mixture was extracted three times with dichloromethane (15 mL). The organic phase was combined, washed once with saturated sodium chloride (15 mL), and dried over anhydrous sodium sulfate. The mixture was concentrated under reduced pressure, and the obtained residue was purified by column chromatography (dichloromethane:methanol = 100:1-80:1-50:1-20:1-15:1) to obtain 80 mg of compound T6, with a yield of 48.2%. NMR analysis was as follows:

¹H NMR: (300 MHz, DMSO-d₆) δ 7.92 (s, 1H), 7.23 (s, 1H), 6.53 (s, 2H), 6.25 (s, 1H), 4.64 (t, J = 8.6 Hz, 2H), 3.24 (t, J = 8.6 Hz, 2H), 4.75 (m, 1H), 3.88-3.70 (m, 4H), 3.69-3.52 (m, 6H), 2.11-2.00 (m, 2H), 1.88-1.80 (m, 2H).

MS (C₂₀H₂₄N₄O₃): 369.21 [M+H]⁺.

### Example 21: Synthesis of Compound T7

The synthesis of the starting material T7-X was performed by reference to the synthetic method for T1-X.

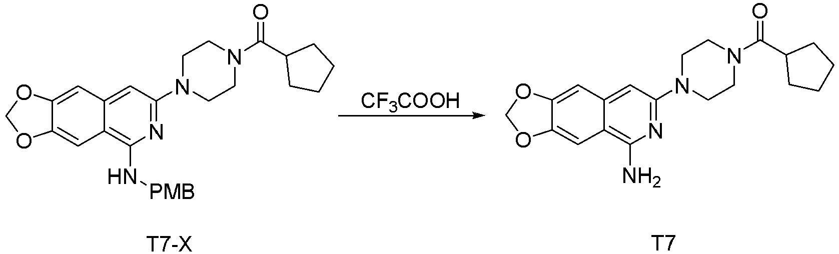

115 mg (0.24 mmol) of intermediate T7-X and 1.2 mL of trifluoroacetic acid were added to a 4 mL reaction tube. The obtained reaction mixture was stirred in an oil bath at 45°C for 3 hours, and the solvent was removed by rotary evaporation under vacuum. 15 mL of saturated NaHCO₃ solution was added to the obtained residue, and the mixture was extracted three times with dichloromethane (15 mL). The organic phase was combined, washed once with saturated sodium chloride (15 mL), and dried over anhydrous sodium sulfate. The mixture was concentrated under reduced pressure, and the obtained residue was purified by column chromatography (dichloromethane:methanol = 100:1-80:1-50:1-20:1) to obtain 34 mg of compound T7, with a yield of 39.2%. NMR analysis was as follows:

¹H NMR: (300 MHz, DMSO-d₆) δ 7.58 (s, 1H), 7.00 (s, 1H), 6.82-6.76 (m, 2H), 6.21 (s, 1H), 6.11 (s, 2H), 3.61-3.60 (m, 4H), 3.30-3.25 (m, 4H), 3.08-2.98 (m, 1H), 1.79-1.52 (m, 8H).

MS (C₂₀H₂₄N₄O₃): 369.22 [M+H]⁺.

### Example 22: Synthesis of Compound T8

The synthesis of the starting material T8-X was performed by reference to the synthetic method for T1-X.

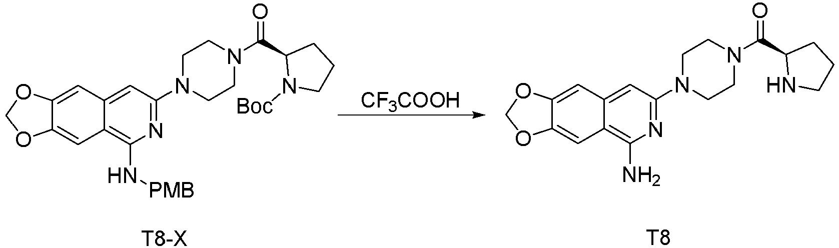

79 mg (0.13 mmol) of intermediate T8-X and 1.6 mL of trifluoroacetic acid were added to a 4 mL reaction tube, and the obtained reaction mixture was stirred and reacted in an oil bath at 45°C for 3 hours. The solvent was removed by rotary evaporation under vacuum. 15 mL of saturated NaHCO₃ solution was added to the obtained residue, and the mixture was extracted three times with dichloromethane (15 mL). The organic phase was combined, washed once with saturated sodium chloride (15 mL), and dried over anhydrous sodium sulfate. The mixture was concentrated under reduced pressure, and the obtained residue was purified by column chromatography (dichloromethane:methanol = 100:1-80:1-50:1-20:1-8:1) to obtain 34 mg of compound T8, with a yield of 68.7%. NMR analysis was as follows:

¹H NMR: (300 MHz, DMSO-d₆) δ 9.72 (brs, 1H), 8.53 (brs, 1H), 7.53 (s, 1H), 6.96 (s, 1H), 6.17 (s, 1H), 6.08 (s, 2H), 4.67 (m, 1H), 3.71-3.61 (m, 4H), 3.39-3.18 (m, 7H), 2.44-2.36 (m, 1H), 1.95-1.81 (m, 3H).

MS (C₁₉H₂₃N₅O₃): 370.27 [M+H]⁺.

### Example 23: Synthesis of Compound T9

The synthesis of the starting material T9-X was performed by reference to the synthetic method for T1-X.

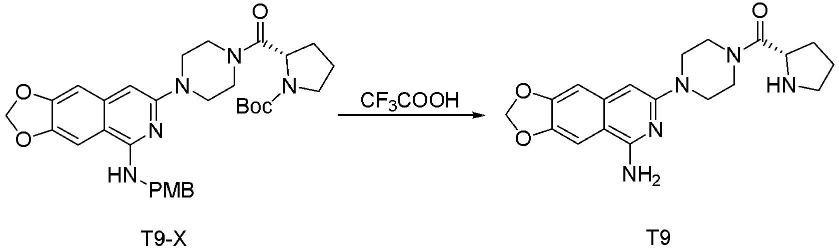

120 mg (0.20 mmol) of intermediate T9-X and 2.4 mL of trifluoroacetic acid were added to a 4 mL reaction tube, and the obtained reaction mixture was stirred and reacted in an oil bath at 45°C for 3 hours. The solvent was removed by rotary evaporation under vacuum. 15 mL of saturated NaHCO₃ solution was added to the obtained residue, and the mixture was extracted three times with dichloromethane (15 mL). The organic phase was combined, washed once with saturated sodium chloride (15 mL), and dried over anhydrous sodium sulfate. The mixture was concentrated under reduced pressure, and the obtained residue was purified by column chromatography (dichloromethane:methanol = 100:1-80:1-50:1-20:1-8:1) to obtain 55 mg of compound T9, with a yield of 73.3%. NMR analysis was as follows:

¹H NMR: (300 MHz, DMSO-d₆) δ 9.69 (brs, 1H), 8.55 (brs, 1H), 7.55 (s, 1H), 6.88 (s, 1H), 6.22 (s, 1H), 6.11 (s, 2H), 4.61 (m, 1H), 3.80-3.65 (m, 4H), 3.45-3.21 (m, 7H), 2.52-2.33 (m, 1H), 2.00-1.80 (m, 3H).

MS (C₁₉H₂₃N₅O₃): 370.25 [M+H]⁺.

### Example 24: Synthesis of Compound T10

The synthesis of the starting material T10-X was performed by reference to the synthetic method for T1-X.

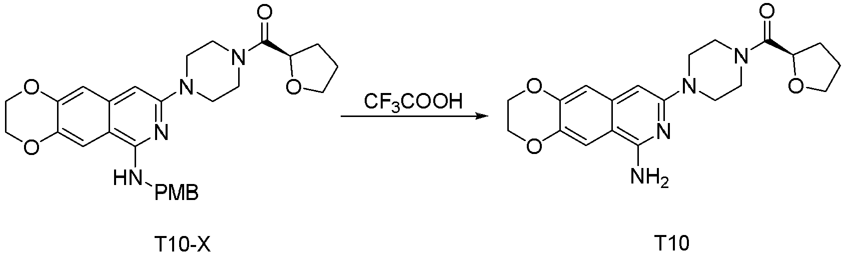

170 mg (0.34 mmol) of intermediate T10-X and 1.7 mL of trifluoroacetic acid were added to a 4 mL reaction tube, and the obtained reaction mixture was stirred and reacted in an oil bath at 45°C for 3 hours. The solvent was removed by rotary evaporation under vacuum. 15 mL of saturated NaHCO₃ solution was added to the obtained residue, and the mixture was extracted three times with dichloromethane (15 mL). The organic phase was combined, washed once with saturated sodium chloride (15 mL), and dried over anhydrous sodium sulfate. The mixture was concentrated under reduced pressure and the obtained residue was purified by column chromatography (dichloromethane:methanol = 100:1-80:1-50:1-20:1-10:1) to obtain 48 mg of compound T10, with a yield of 37.1%. NMR analysis was as follows:

¹H NMR: (300 MHz, DMSO-d₆) δ 8.50 (br, 2H), 7.91 (s, 1H), 7.16 (s, 1H), 6.33 (s, 1H), 4.75-4.72 (m, 1H), 4.42-4.35 (m, 4H), 3.83-3.76 (m, 2H), 3.73-3.56 (m, 4H), 3.26-3.10 (m, 4H), 2.14-1.97 (m, 2H), 1.90-1.79 (m, 2H).

MS (C₂₀H₂₄N₄O₄): 385.05 [M+H]⁺.

### Example 25: Synthesis of Compound T11

The synthesis of the starting material T11-X was performed by reference to the synthetic method for T1-X.

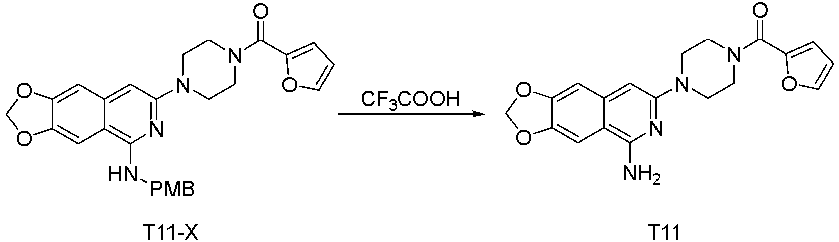

77 mg (0.16 mmol) of intermediate T11-X and 1.5 mL of trifluoroacetic acid were added to a 4 mL reaction tube. The obtained reaction mixture was stirred and reacted in an oil bath at 45°C for 3 hours, and the solvent was removed by rotary evaporation under vacuum. 15 mL of saturated NaHCO₃ solution was added to the obtained residue, and the mixture was extracted three times with dichloromethane (15 mL). The organic phase was combined, washed once with saturated sodium chloride (15 mL), and dried over anhydrous sodium sulfate. The mixture was concentrated under reduced pressure, and the obtained residue was purified by column chromatography (dichloromethane:methanol = 100:1-80:1-50:1-20:1-10:1) to obtain 38 mg of compound T11, with a yield of 65.5%. NMR analysis was as follows:

¹H NMR: (300 MHz, DMSO-d₆) δ 7.87-7.86 (m, 1H), 7.56 (s, 1H), 7.21-7.18 (m, 1H), 7.05-7.04 (m, 1H), 6.97 (s, 1H), 6.93-6.79 (m, 1H), 6.65-6.64 (m, 1H), 6.19 (s, 1H), 6.10 (s, 2H), 3.80 (br, 4H), 3.41-3.39 (m, 4H).

MS (C₁₉H₁₈N₄O₄): 367.23 [M+H]⁺.

### Example 26: Preparation of Compound T1 Trifluoroacetate

250 mg (0.51 mmol) of intermediate T1-X and 2.5 mL of trifluoroacetic acid were added to a 4 mL reaction tube. The obtained reaction mixture was stirred and reacted in an oil bath at 45°C for 3 hours, and the solvent was removed by rotary evaporation under vacuum. The obtained residue was washed twice with methanol (2.5 mL), filtered, and dried under vacuum at room temperature to obtain 112 mg of compound T1 trifluoroacetate, with a yield of 45.3%. NMR analysis was as follows:

¹H NMR: (300 MHz, DMSO-d₆) δ 7.44 (s, 1H), 6.88 (s, 1H), 6.32 (brs, 2H), 6.08 (s, 1H), 6.03 (s, 2H), 4.71 (m, 1H), 3.82-3.70 (m, 4H), 3.66-3.55 (m, 6H), 2.08-1.99 (m, 2H), 1.88-1.80 (m, 2H).

MS (C₂₁H₂₃F₃N₄O₆): 371.14 [M+H-TFA]⁺.

### Example 27: Preparation of Compound T1 Hydrochloride

120 mg of compound T1 and 2.4 mL of methanol were added to a 4 mL reaction tube. The obtained mixture was stirred and cooled in an ice-water bath. 0.054 mL of concentrated hydrochloric acid was added to the mixture. The obtained mixture was stirred in an ice bath for 1 hour, and then filtered. The filter cake was washed with ice-cold methanol and dried under vacuum to obtain 103 mg of compound T1 hydrochloride, with a yield of 78.0%. NMR analysis was as follows:

¹H NMR: (300 MHz, DMSO-d₆) δ 8.78 (brs, 3H), 7.48 (s, 1H), 6.91 (s, 1H), 6.11 (s, 1H), 6.05 (s, 2H), 4.74 (m, 1H), 3.84-3.71 (m, 4H), 3.70-3.57 (m, 6H), 2.10-2.01 (m, 2H), 1.90-1.80 (m, 2H).

MS (C₁₉H₂₃ClN₄O₄): 371.19 [M+H-HCl]⁺.

### Example 28: Preparation of Compound T1 Sulfate

200 mg of compound T1 and 4.0 mL of methanol were added to a 4 mL reaction tube. The obtained mixture was stirred and cooled in an ice-water bath. 0.058 mL of concentrated sulfuric acid was added to the mixture. The obtained mixture was stirred in an ice bath for 1 hour, and then filtered. The filter cake was washed with ice-cold methanol and dried under vacuum to obtain 213 mg of compound T1 sulfate, with a yield of 84.2%. ¹H NMR analysis was as follows:

¹H NMR: (300 MHz, DMSO-d₆) δ 8.89 (brs, 2H), 7.46 (s, 1H), 6.90 (s, 1H), 6.13 (s, 1H), 6.06 (s, 2H), 4.75 (m, 1H), 3.83-3.69 (m, 4H), 3.68-3.55 (m, 6H), 2.10-1.99 (m, 2H), 1.86-1.78 (m, 2H).

MS (C₁₉H₂₄N₄O₈S): 371.25 [M+H-H₂SO₄]⁺.

### Example 29: Determination of Effects of Compounds on Cellular Glucose Metabolism.

Effects of compounds on cellular ATP levels.

### Experimental Conditions:

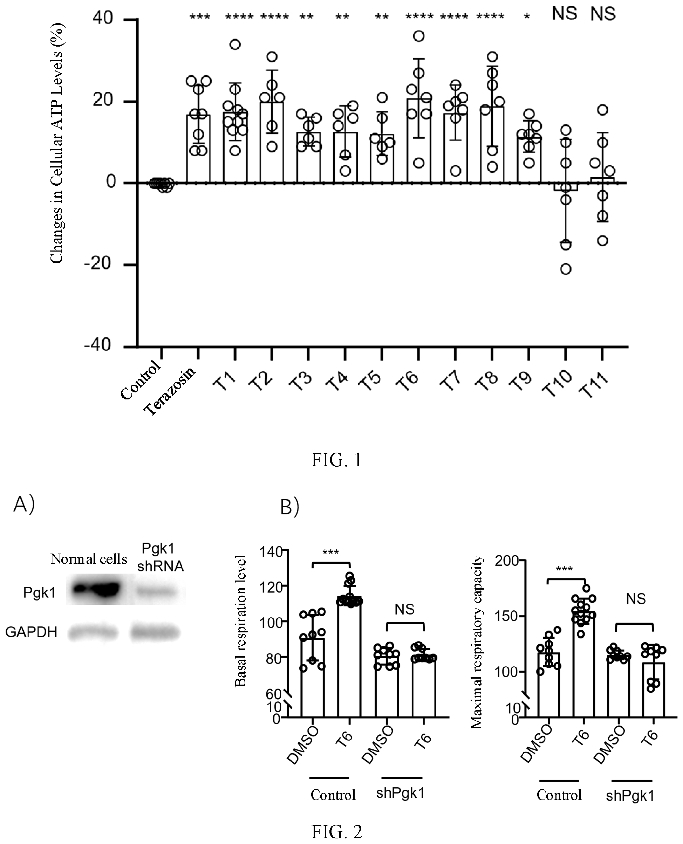

Human Neuroblastoma Cell Line (SK-N-SH) is a human-derived neuroblastoma cell line. SK-N-SH cells were seeded in a 6-well plate at a density of 6×10⁴ cells per well. The cells were cultured for 12 hours under 5% O₂. Various drugs at a concentration of 10 µM were added. After 48 hours, the cells were harvested. Celluar ATP levels were measured using the Promega ATP assay kit. The experiment was repeated 6 times to 10 times. The experimental results were illustrated in FIG. 1. The ATP level in the untreated group (control) was defined as 1, and the terazosin treatment group was defined as the positive control. T1 to T11 were the experimental groups.

Experimental Results: T1 to T9 each exhibited an activity in increasing cellular ATP, while T10 and T11 exhibited no such activity of increasing cellular ATP.

### Example 30: Determination of Affinities of Compounds for Pgk1 Protein

### Experimental Conditions:

Purified Pgk1 protein (with a concentration of 1 mg/mL) was immobilized on a Biacore T200 chip. After confirmation of protein coupling, different concentrations of the drugs were injected. The obtained curves can calculate parameters such as affinity and Rmax.

The experimental results were shown in Table 1.

**Table 1**

| Analyzed Drug | Affinity (µM) | Rmax (RU) |
|---|---|---|
| Terazosin | 1.4 | 62 |
| T1 | 496.4 | 116 |
| T2 | 448.8 | 122 |
| T3 | 683.8 | 93 |
| T4 | 725.4 | 75 |
| T5 | 272.8 | 86 |
| T6 | 375.5 | 106 |
| T7 | 878.1 | 159 |
| T8 | 473.4 | 87 |
| T9 | 811.3 | 87 |
| T10 | >1000 | None |
| T11 | >1000 | None |
| ADP | 337.4 | 35 |
| Terazosin+ADP | 2.9 | 62 |
| T6+ADP | >1000 | None |

Experimental Results: Table 1 listed the affinities of various drugs for Pgk1. Terazosin exhibited the highest affinity, with a value of 1.4 µM. T1 to T9 all showed affinity for Pgk1 protein, but their affinities for Pgk1 protein were much lower than that of terazosin. No affinity for the Pgk1 protein was detected for T10 and T11 (KD value greater than 1000 µM). Rmax (RU) referred to the amount of small-molecule drugs bound to the target protein when the affinity was detected. Our published studies have shown that terazosin promoted the dissociation of ATP from the Pgk1 protein by interfering with ATP, relieving the inhibitory effect of ATP on the Pgk1 enzymatic reaction. A higher Rmax value indicated a greater potential to interfere with the dissociation of ATP. ADP was a substrate of Pgk1, with a KD value of 337.4. When detecting the affinity of terazosin, the addition of saturated ADP can reduce the KD value of terazosin from 1.4 µM to 2.9 µM, indicating that the binding site of terazosin on the Pgk1 protein overlaps with that of ADP/ATP. The KD value of T6 was also significantly reduced after the addition of ADP, indicating that the binding of T6 to Pgk1 can be interfered by ADP and that the binding site of T6 on the Pgk1 protein was similar to that of terazosin. This result revealed that multiple isoquinoline derivatives exhibited affinity for Pgk1, albeit weaker than that of terazosin.

### Example 31: Determination of Effects of Compounds on Mitochondrial Respiration

### Experimental Conditions:

Cellular mitochondrial stress was detected using a Seahorse XFe analyzer (XFe-24, Seahorse Bioscience, Billerica, MA, UAS) to measure the oxygen consumption rate (OCR). SH-SKN cell lines were seeded in a 24-well plate compatible with the Seahorse XFe analyzer. After 24 hours, cells were treated with DMSO (0.01%) or various drugs (10 µM). Cellular mitochondrial stress was detected 12 hours after drug treatment. For the mitochondrial stress test, 97.2 mL of Seahorse basal medium (Seahorse Bioscience, Billerica, MA, UAS) was taken and heated in a water bath to 37°C, and 1 mL of a stock solution was added. The stock solution was prepared by mixing the following solutions: 1 mL of 200 mM L-glutamine solution (Sigma), 1 mL of 100 mM pyruvate solution (Sigma), and 800 µL of 2.5 M glucose solution, and the pH of the stock solution was adjusted to 7.4 with 1 M NaOH solution. Before detection, the probe plate was taken out, and 1 mL of XF Carlibrant solution (Seahorse Bioscience) was added to the probe plate. The plate was then incubated overnight in a CO₂-free constant temperature incubator at 37°C. After drug treatment, the cells were removed and washed twice with preheated Seahorse basal medium at 37°C, ensuring that 500 µL of Seahorse basal medium remained in each well. The cells were incubated in a CO₂-free constant temperature incubator at 37°C for 1 hour. During this period, the probe plate was removed from the incubator, and oligomycin, FCCP, and Rotenone/antimycin A were added into the probe plate. Subsequently, the probe plate was placed into the Seahorse XFe Analyzer for calibration. All drugs were used at 10 µM. The experiment was repeated 3 times to 5 times.

The experimental results were shown in Table 2.

**Table 2**

| Analyzed Drug | Increase in Maximal Mitochondrial Respiration (%) |
|---|---|
| Terazosin | 16.3±2.7* |
| T1 | 22.1±3.1** |
| T2 | 17.8±2.2* |
| T3 | 15.3±0.9* |
| T4 | 18.1±0.7* |
| T5 | 16.6±0.8* |
| T6 | 26.3±3.6*** |
| T7 | 21.5±4.1* |
| T8 | 19.2±3.8** |
| T9 | 20.1±0.8** |
| T10 | 6.3±0.4 |
| T11 | 8.6±0.6 |

Table 2 listed the effects of the drugs on basal cellular respiration, with the control group (DMSO treatment) as 1. Statistically significant differences were marked with asterisks. The results showed that terazosin and T1 to T9 all exhibited the activity of enhancing mitochondrial respiration, while T10 and T11 exhibited no activity of activating mitochondrion. These results were consistent with those in FIG. 1, indicating that the newly synthesized isoquinoline derivatives (T1 to T9) exhibited the activity of activating Pgk1 and increasing mitochondrial respiration.

### Example 32: Determination of whether Compounds Produce Therapeutic Effects via Pgk1

### Experimental Conditions:

To further determine that the newly synthesized isoquinoline derivatives produced the activity in activating mitochondrion through targeting Pgk1, a stable Pgk1-knockdown SK-N-SH cell line was constructed in the present disclosure. The target sequence of human Pgk1 was: GAGCTAAAGTTGCAGACAA. A BamHI restriction site was added to the 5' end of the knockdown sequence, an XbaI restriction site was added to the 3' end of the knockdown sequence, and a loop was inserted in the middle to synthesize the top strand 5'-gatccGAGCTAAAGTTGCAGACAATTCAAGAGATTGTCTGCAACTTTAGCTCt -3', and the bottom strand 5'-ctagaGAGCTAAAGTTGCAGACAATCTCTTGAATTGTCTGCAACTTTAGCTCg-3'. The two DNA oligos were phosphorylated at the 5' end and then inserted into the plenti-u6 vector. The obtained Lenti-shPgk1 vector was transfected into HEK293T cells with pCAG-dR8.9 and pCMV-VSV-G at a molar ratio of 1:1:1. The supernatant was collected after 72 hours. The obtained supernatant was subjected to ultracentrifugation to obtain viral pellets, which were resuspended in 1/100 volume of PBS. After standing for 24 hours, the viral suspension was used to infect SH-SKN cells pretreated with polybrene. 48 hours after infection, the virus-infected cells were subjected to limited dilution and seeded into a 96-well plate to obtain monoclonal cell lines. The obtained monoclonal cell lines were further expanded and cultured. The knockdown efficiency of Pgk1 was further verified by western blot. The results were illustrated in FIG. 2, in which A represented the Western blot assay and B represented the mitochondrial respiratory physiology assay (seahorse assay). FIG. 2A showed that Pgk1 protein was significantly knocked down in the stable shPgk1-expressing cell line. In this Western blot assay, GAPDH was used as a loading control to guarantee the same loading amount. FIG. 2B showed the effect of T6 on mitochondrial respiration using this cell line. The experimental conditions and administration time for Seahorse were the same as those in Example 31. The results showed that T6 (10 µM) activated both the basal respiratory rate and maximum respiratory rate in normal cells (control); however, this activity was absent in shPgk1 knockdown cells. This indicated that the activation of mitochondrial metabolism by T6 depended on Pgk1. Other compounds also showed similar results.

### Example 33: Determination of Dose-effect Relationships of Compounds in In Vitro Biochemical Assays

### Experimental Conditions:

Human Pgk1 cDNA was cloned into the pEASYTM-E2 vector (TransGen Biotech) containing a His tag. Protein expression was induced in BL21(DE3) chemocompetent cells (TransGen Biotech) with 0.5 mM IPTG. After lysing cells in extraction buffer (20 mM sodium phosphate, 500 mM NaCl, 5 mM imidazole, 5% glycerol, protease inhibitor cocktail, pH 7.9), the supernatant was purified with nickel beads (AdarBiotech). The His-tagged Pgk1 was eluted with the elution buffer (20 mM sodium phosphate, 500 mM NaCl, 80 mM imidazole, protease inhibitor cocktail, pH 7.9). The activity of Pgk1 can be measured by monitoring the rate of NADH production, as NADH accumulated as the reaction proceeded. Purified Pgkl-His protein (2 µg/mL) was added to the reaction buffer (20 mM Tris, 100 mM NaCl, 2 mM DTT, 0.1 mM MgSO₄, 10 mM NaHPO₄, pH 8.6), and the substrates (1.6 mM GAP, 1 mM β-NAD, 1 mM ADP, 20 ng/µL GAPDH) was added. Different concentrations of terazosin (positive control) and T6 were added to the reaction system. After reaction for 10 minutes at room temperature, the absorbance wavelength at 339 nm was measured using a microplate reader (Molecular Devices, SpectraMax i3x), and this wavelength corresponds to NADH levels.

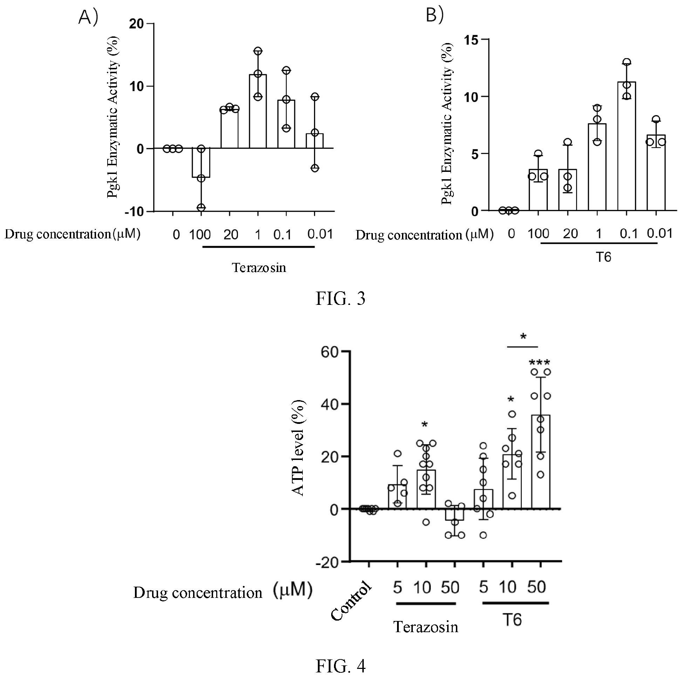

The experimental results were illustrated in FIG. 3, in which A represented the effect of terazosin on Pgk1 enzymatic activity in cells, and B represented the effect of compound T6 on Pgk1 enzymatic activity in cells.

As illustrated in FIG. 3A, as a positive control, the effects of terazosin at concentrations of 100 µM, 20 µM, 1 µM, 0.1 µM, and 0.01 µM on Pgk1 enzymatic activity were tested. The results showed that a high concentration (100 µM) of terazosin can inhibit Pgk1 enzymatic activity, while a low concentration (1 µM) of terazosin exhibited the best activation effect on Pgk1. FIG. 3B compared the effects of different concentrations of T6 on Pgk1, finding that none of the concentrations can inhibit Pgk1. The optimal concentration for activating Pgk1 was 0.1 µM. This result indicated that T6 did not pose a risk of inhibiting Pgk1, and it was unlikely to reduce ATP levels in brain tissue in patients, reducing the risk associated with quinazoline drugs. Similar results were observed for T1 to T9.

### Example 34: Determination of Dose-effect Relationships of Compounds in Cultured Cells

### Experimental Conditions:

SK-N-SH cells were seeded in a 6-well plate at a density of 6×10⁴ per well. Cells were cultured for 12 hours under 5% O₂, and the drug was added. Cells were harvested after 48 hours. Cellular ATP levels were measured using a Promega ATP assay kit. The experiment was repeated 5 times to 7 times.

The experimental results were illustrated in FIG. 4.

As illustrated in FIG. 4, the positive controls terazosin (TZ) and T6 were both administered at doses of 5 µM, 10 µM, and 50 µM. The optimal dose of TZ was 10 µM, and the optimal dose of T6 was 50 µM. However, both TZ and T6 at a concentration of 50 µM exhibited a tendency to inhibit cell growth, the biologically effective concentration of T6 should be close to 10 µM.

### Example 35: Determination of Effects of Compounds on Blood Glucose in Mice In Vivo

### Experimental Conditions:

Wild-type mice (C57BL/6) aged 3 months to 4 months under normal feeding conditions were intraperitoneally injected with different drugs at 0.1 mg/kg. Mice injected with normal saline served as the control group. Mice were fasted for 6 hours after administration. Blood was collected from the tail of each mouse to measure blood glucose levels. Each group consisted of 9 mice.

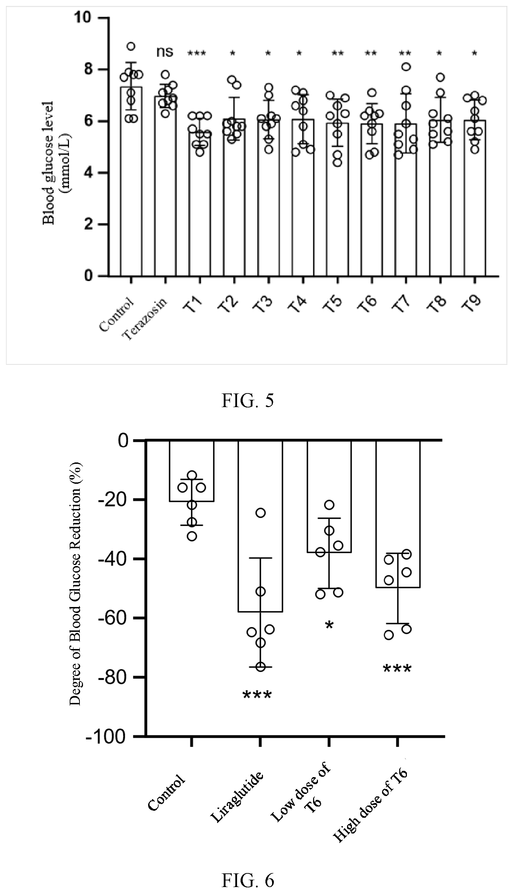

The experimental results were illustrated in FIG. 5.

As illustrated in FIG. 5, the terazosin (TZ) group did not exhibit a hypoglycemic effect. However, T1 to T9 each exhibited a significant hypoglycemic effect. This indicated that this novel class of isoquinoline derivatives had higher activity in activating glucose metabolism.

### Example 36: Determination of Hypoglycemic Effects of Compounds in Diabetic Model Mice

### Experimental Conditions:

Gene knockout db/db mice are a classic model of hyperglycemia and obesity. Under normal feeding conditions, mice aged 3 months or older developed hyperglycemia and obesity. After the mice were provided with only drinking water (fasted) for 6 hours, blood was collected from the tail of the mice to measure blood glucose values. Subsequently, different drugs, including saline (control), liraglutide at 0.6 mg/Kg, low-concentration T6 (T6 low, 0.1 mg/Kg), and high-concentration T6 (T6 high, 0.5 mg/Kg), were injected intraperitoneally. 4 hours later, blood was collected from the tail of the mice to measure the blood glucose levels. Each group consisted of 6 mice. Blood glucose levels before administration (V_{before}) and after administration (V_{after}) were obtained for each mouse. The effect of the drug on blood glucose (%) was calculated as follows:
$\left({V}_{before}-{V}_{after}\right) \times 100 / {V}_{before} .$

The experimental results were illustrated in FIG. 6.

As illustrated in FIG. 6, liraglutide, as a positive control, exhibited the optimal hypoglycemic effect. T6 at both low concentration (0.1 mg/kg) and high concentration (0.5 mg/kg) also showed significant hypoglycemic effects. This result further confirmed that intraperitoneal injection of the novel isoquinoline derivatives exerted hypoglycemic effects.

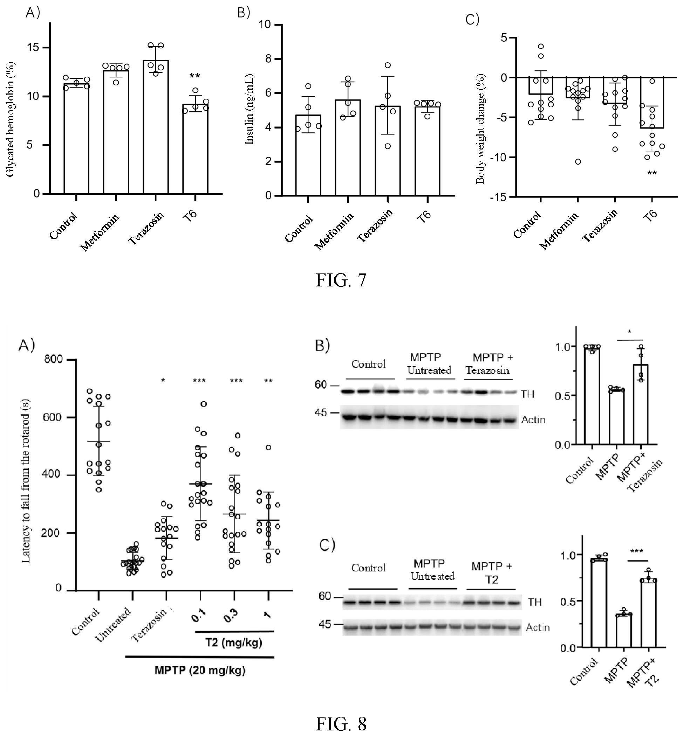

The hypoglycemic effect of the novel isoquinoline derivatives via intraperitoneal injection lasted no more than 6 hours. To determine the long-term hypoglycemic effect of T6, T6 was added to drinking water and administered to mice continuously for one week. FIG. 7 showed the long-term effect via drinking water administration on blood glucose levels in mice.

### Example 37: Determination of Long-Term Hypoglycemic and Weight-Loss Effects of Compounds in Diabetic Model Mice

### Experimental Conditions:

Gene knockout db/db mice under normal feeding were adminstered with metformin (350 mg/kg), terazosin (0.3 mg/kg), or T6 (1.5 mg/kg) in drinking water. After 7 days of treatment, changes in glycated hemoglobin (HbA1c) level, insulin level in mouse blood, and body weight, were measured.

The experimental results were illustrated in FIG. 7, in which A represented glycated hemoglobin level, B represented insulin level, and C represented changes in mouse body weight.

As illustrated in FIG. 7, compared with the control group, only the T6 treatment group showed a decrease in glycated hemoglobin (HbA1c) level. Insulin level remained unchanged in all treatment groups. The T6 treatment group showed a significant decrease in body weight, which was distinct from the effects observed in the terazosin and metformin treatment groups. The above-described results indicated that, with long-term oral administration, T6 exerted a better hypoglycemic effect than that of terazosin and metformin and also exerted a weight-loss effect.

### Example 38: Determination of Effects of Compounds on Dopaminergic Neurons in an MPTP-induced Mouse Model of Parkinson's Disease

### Experimental Conditions:

Male mice aged 3 months were used and fed normally. The acute MPTP modeling method (see Sonsalla PK, Heikkila RE. The influence of dose and dosing interval on MPTP-induced dopaminergic neurotoxicity in mice. Eur J Pharmacol 1986; 129(3): 339-45.) was employed. MPTP was injected intraperitoneally four times in total at a dose of 20 mg/kg body weight per injection, with a 2-hour interval between each injection. MPTP was dissolved in normal saline, and the control group received an equal dose of normal saline. T2 was dissolved in DMSO and diluted with normal saline to the desired concentration. Accordingly, equal doses of DMSO and normal saline were administered during the treatment. On the day of MPTP model establishment, mice were administered either T2 (treatment group) or DMSO (control group). Subsequently, mice in all groups were administered the corresponding drugs daily. Mouse behavior was tested using a rotarod (E103, UGO BASILE). First, mice were pre-trained for two consecutive days at a constant speed of 15 revolutions per minute (15 rpm) until they could stay on the rod for more than 60 seconds. On day 7 after MPTP injection, mice were tested on the rotarod in an accelerating mode (2 rpm to 45 rpm). The latency to fall from the rotarod was recorded, and the behavior was monitored by a camera. After the experiment, the mice were sacrificed, and the striatal brain tissue was harvested for Western blot assay. Tyrosine hydroxylase (TH) is a protein specifically expressed in dopaminergic neurons. Therefore, whether dopaminergic neurons were damaged can be determined by Western blot assay using an anti-TH antibody. Terazosin (TZ, 0.1 mg/Kg) was set as a positive control, and three doses of T2 were tested: 0.1 mg/Kg, 0.3 mg/Kg, and 1 mg/Kg.

The experimental results were illustrated in FIG. 8. A represented an effect of each drug on the motor ability of MPTP-induced Parkinson's disease model mice, B represented an effect of terazosin on the tyrosine hydroxylase level in MPTP-induced Parkinson's disease model mice, and C represented an effect of compound T2 at a dose of 0.1 mg/Kg on the tyrosine hydroxylase level in MPTP-induced Parkinson's disease model mice.

As illustrated in FIG. 8A, treatment with terazosin (TZ) improved the motor ability of mice under MPTP-induced injury. All three doses of T2 all exerted protective effects. In FIG. 8B, the tyrosine hydroxylase protein levels are compared among the control group, MPTP-treated group, and the terazosin-treated group. Striatal proteins were randomly selected from four mice in each group for Western blot assay, and the statistical results were shown on the right. These results indicated that terazosin exerted a protective effect on dopaminergic neurons, and this group served as a positive control. FIG. 8C represented dopaminergic neurons, and the method used was similar to that in FIG. 8B. These results indicated that T2 exerted a protective effect on dopaminergic neurons.

The embodiments described above are merely illustrative of preferred embodiments of the present disclosure and are not intended to limit the scope of the present disclosure. Without departing from the spirit of the present disclosure, various modifications and improvements made to the technical solutions of the present disclosure by those skilled in the art should fall within the protection scope defined by the claims of the present disclosure.

## Claims

1. An isoquinoline derivative represented by a general structural formula as follows: 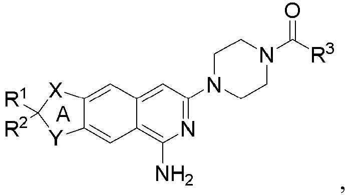 wherein:
ring A represents an optionally substituted five-membered oxygen-containing heterocycle;
X is O or C, Y is O or C, and at least one of X and Y is O;
R¹ represents hydrogen or methyl;
R² represents hydrogen or methyl; and
R³ is five-membered cycloalkyl, or five-membered heterocyclic alkyl containing one heteroatom selected from an oxygen atom or a nitrogen atom,
wherein the isoquinoline derivative has any one of the following specific structures:

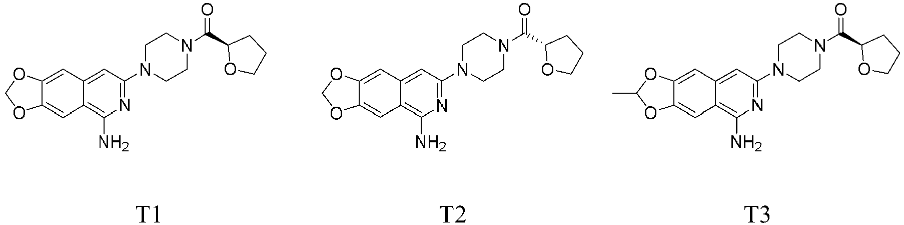

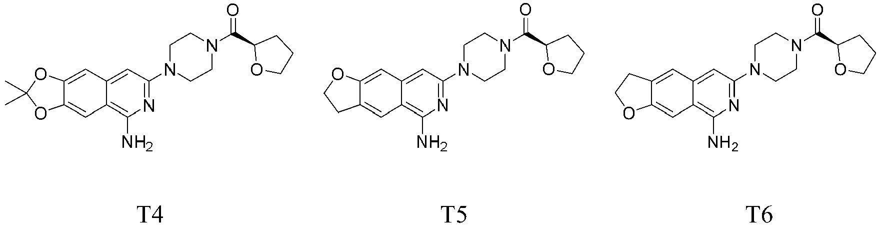

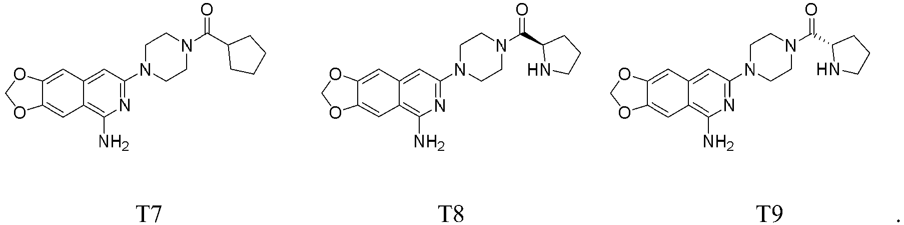

2. A pharmaceutically acceptable salt of the isoquinoline derivative according to claim 1, comprising hydrochloride, sulfate, hydrobromide, nitrate, trifluoroacetate, acetate, phosphate, benzenesulfonate, p-toluenesulfonate, methanesulfonate, oxalate, tartrate, fumarate, maleate, succinate, malate, benzoate, citrate, or lactate.

3. An isoquinoline derivative formulation, comprising, as an active ingredient:
the isoquinoline derivative according to claim 1; and/or
the pharmaceutically acceptable salt of the isoquinoline derivative according to claim 2,
wherein the formulation is respective pharmaceutical formulations prepared from the isoquinoline derivative or pharmaceutically acceptable salt thereof with a pharmaceutically acceptable excipient, such as water, alcohol, vegetable oil, starch, microcrystalline cellulose, lactose, gelatin, magnesium stearate, sodium alginate, maltodextrin, talc, crospovidone, gum, petrolatum, and the like.

4. A phosphoglycerate kinase activator, wherein the activator is the isoquinoline derivative according to claim 1, the pharmaceutically acceptable salt of the isoquinoline derivative according to claim 2, or the isoquinoline derivative formulation according to claim 3.

5. A medicament for treating or preventing a neurodegenerative disease, wherein the medicament is the isoquinoline derivative according to claim 1, the pharmaceutically acceptable salt of the isoquinoline derivative according to claim 2, or the isoquinoline derivative formulation according to claim 3.

6. A medicament for treating or preventing a metabolic disease, wherein the medicament is the isoquinoline derivative according to claim 1, the pharmaceutically acceptable salt of the isoquinoline derivative according to claim 2, or the isoquinoline derivative formulation according to claim 3.

7. A method for preparing the isoquinoline derivative according to claim 1, the method comprising one of the following synthetic routes or a combination thereof:
synthetic route A:
adding malonic acid and piperidine to a solution of compound I for reaction, to obtain compound II;
reacting compound II in the presence of a catalyst under a H₂ atmosphere to obtain compound III;
reacting compound III with an oxalyl chloride to generate an acyl chloride, then reacting under catalysis of aluminum trichloride to obtain compound IV;
reacting compound IV with isoamyl nitrite under an acidic condition to obtain compound V;
reacting compound V with phosphorus oxychloride to obtain compound VI;
reacting compound VI with an amino protecting reagent in N-methylpyrrolidone to obtain compound VII;
reacting compound VII, compound XI, dioxane, Pd₂(dba)₃, RuPhos, and cesium carbonate together to obtain compound VIII;
deprotecting compound VIII to remove a Boc protecting group to obtain compound IX;
reacting compound IX with R³-COOH to obtain compound X; and
reacting compound X with trifluoroacetic acid to remove an amino protecting group to obtain isoquinoline derivative compound T, the reaction route A being as follows: 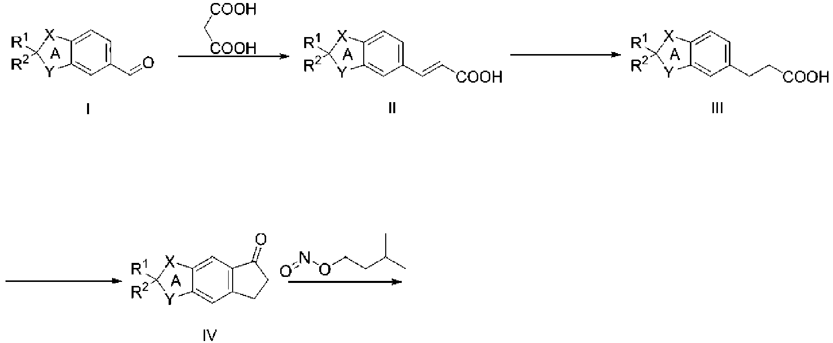 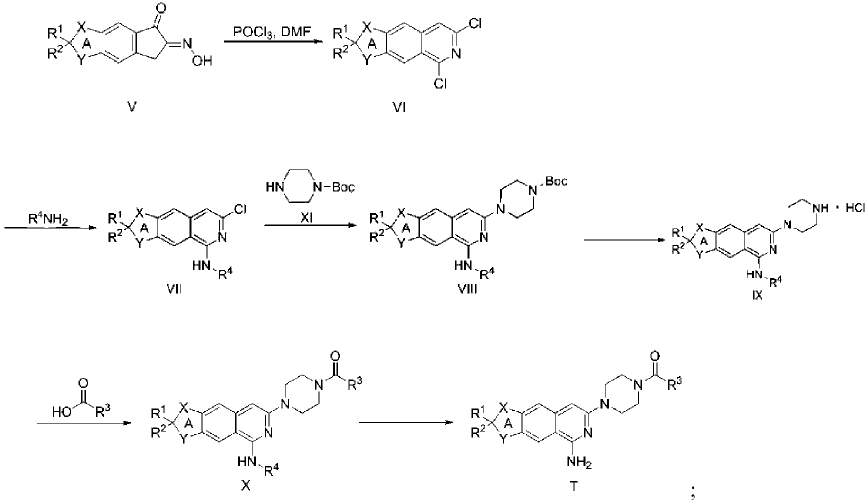
synthetic route B:
reacting compound XI with R³-COOH, O-(7-azabenzotriazol-1-yl)-N,N,N',N'-tetramethyluronium hexafluorophosphate, and N,N-diisopropylethylamine in a solvent to obtain compound XII;
deprotecting compound XII to remove a Boc protecting group to obtain compound XIII;
reacting compound XIII with compound VII obtained according to synthetic route A, dioxane, Pd₂(dba)₃, RuPhos, and cesium carbonate together to obtain compound X; and
reacting compound X with trifluoroacetic acid to remove an amino protecting group to obtain isoquinoline derivative compound T; the reaction route B being as follows: 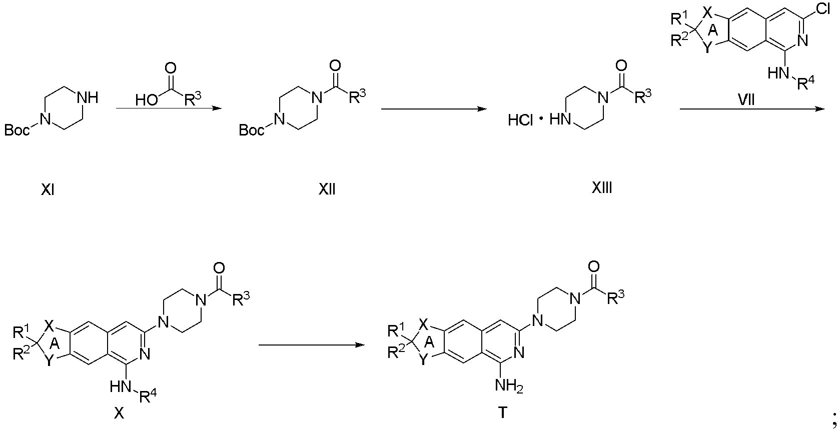 and
synthetic route C:
reacting compound VI obtained according to synthetic route A with ammonia water to obtain compound XIV;
reacting compound XIV with an amino protecting reagent to obtain compound VII; and
treating compound VII according to a method of synthetic route A to obtain isoquinoline derivative Compound T; the reaction route C being as follows: 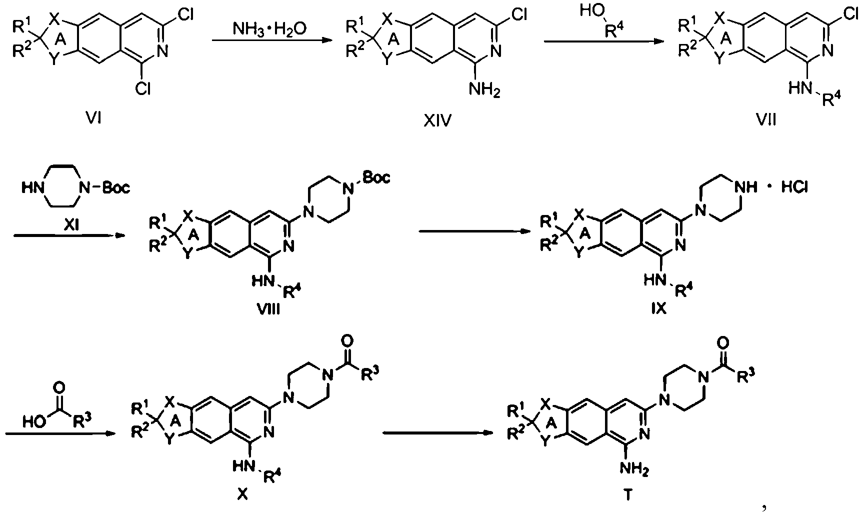
wherein R⁴ of compounds involved in synthetic route A, synthetic route B, and synthetic route C is an amino protecting group.
